# EUROPÄISCHE PATENTANMELDUNG

(11) **EP 1 306 382 A1**
(43) Veröffentlichungstag der Anmeldung: **02.05.2003**
(21) Anmeldenummer: 01125630.2
(22) Anmeldetag: 26.10.2001
(51) Int. Cl.: C07H 21/00, G01N 33/53, C12Q 1/68, A61K 31/7088

(54) **Modifizierte L-Nukleinsäure**

(71) Anmelder: Noxxon Pharma AG, 13355 Berlin (DE)
(72) Erfinder: Klussmann, Sven, 10709 Berlin (DE); Eschgfäller, Bernd, 10781 Berlin (DE)
(74) Vertreter: Bohmann, Armin K., Dr.

(57) **Zusammenfassung**

Die vorliegende Erfindung betrifft eine modifizierte L-Nukleinsäure umfassend einen L-Nukleinsäure-Teil und einen Nicht-L-Nukleinsäure-Teil, wobei der L-Nukleinsäure-Teil mit dem Nicht-L-Nukleinsäure-Teil konjugiert ist und die Konjugation des L-Nukleinsäure-Teils mit dem Nicht-L-Nukleinsäure-Teil zu einer verlangsamten Ausscheidung aus einem Organismus führt, verglichen mit einer L-Nukleinsäure umfassend nur den L-Nukleinsäure-Teil.

SEQUENZPROTOKOLL

<110> NOXXON Pharma AG

<120> Modifizierte L-Nukleinsäure

<130> N 10018 EP

<140>
<141>

<160> 3

<170> PatentIn Ver. 2.1

<210> 1
<211> 67
<212> DNA
<213> Künstliche Sequenz

<220>
<223> Beschreibung der künstlichen Sequenz:
L-Nukleinsäure, die spezifisch an GnRH bindet

<400> 1
CCAAGCTTGC GTAAGCAGTC TCCTCTCAGG GGAGGTTGGG CGGTGCGTAA GCACCGGTTT 60
GCAGGGG 67

<210> 2
<211> 10
<212> DNA
<213> Künstliche Sequenz
<223> Beschreibung der künstlichen Sequenz: modifizierte L-Nukleinsäure

<220>
<221> modifizierte Base
<222> (1)
<223> NH2-Gruppe am 5'-Ende

<400> 2
TATTAGAGAC 10

<210> 3
<211> 10
<212> DNA
<213> Künstliche Sequenz
<223> Beschreibung der künstlichen Sequenz: modifizierte L-Nukleinsäure

<220>
<221> modifizierte Base
<222> (1)
<223> PO4-Gruppe am 5'-Ende

<400> 3
TATTAGAGAC 10

## Beschreibung

Die vorliegende Erfindung betrifft modifizierte L-Nukleinsäuren, deren Verwendung sowie Verfahren zu deren Herstellung.

Die Entwicklung neuer therapeutischer Konzepte greift neben der Verwendung von vergleichsweise kleinen organischen Molekülen zunehmend auch auf monoklonale Antikörper, Peptide und funktionale Nukleinsäuren zurück, d.h. solche Nukleinsäuren, die spezifisch an eine Zielstruktur binden. Typische Vertreter dieser funktionalen Nukleinsäuren sind die sogenannten Aptamere, die gegen eine Vielzahl verschiedener Biomoleküle bereits entwickelt wurden. Dabei wird ausgehend von einer D-Nukleinsäurebibliothek in mehreren Stufen durch in-vitro Selektion ein oder mehrere Nukleinsäuremoleküle, die sogenannten Aptamere, isoliert, die sich durch eine besonders hohe Affinität gegenüber ihrer Zielstruktur auszeichnen. Verfahren zur Herstellung derartiger Aptamere sind beispielsweise beschrieben in der europäischen Patentanmeldung EP 0 533 838.

In der Pharmakologie ist das Problem der Stabilität und biologischen Verfügbarkeit, ausgedrückt als die biologische Halbwertszeit, der verabreichten pharmazeutisch aktiven Wirkstoffe hinlänglich bekannt. Strategien, eine biologische Halbwertszeit zu erreichen, die die optimale Wirkung der verabreichten, pharmazeutisch aktiven Substanzen erlaubt, konzentrieren sich zum einen auf eine geeignete Modifikation der pharmazeutisch aktiven Wirkstoffe und zum anderen auf die Entwicklung geeigneter Darreichungsformen. Im ersteren Falle bestehen erhebliche Beschränkungen dergestalt, dass sichergestellt werden muss, dass die eine erhöhte biologische Halbwertszeit, d.h. Verweildauer im zu behandelnden Organismus, aufweisende Verbindung ihre pharmakologischen Eigenschaften nicht verliert, mit anderen Worten ihre Wirksamkeit sowie das Verursachen möglichst geringer Nebenwirkungen.

Neben den vorstehend genannten Aptameren existieren mit den sogenannten Spiegelmeren eine weitere Form der funktionalen Nukleinsäuren. Auch die Spiegelmere binden spezifisch an eine Zielsequenz, wobei hier jedoch unter Verwendung einer D-Nukleinsäurebibliothek gegen die enantiomere Form des Targets selektiert wird und die daran bindenden D-Nukleinsäuren sodann als L-Nukleinsäuren hergestellt werden und diese infolge der chiralen Reziprozität an das eigentliche Target und nicht an die für den Selektionsprozess verwendete enantiomere Form desselben binden können. Verfahren zur Herstellung derartiger Spiegelmere sind beispielsweise beschrieben in der internationale Patentanmeldung WO 98/08856.

Rein chemisch betrachtet sind Spiegelmere L-Nukleinsäuren, typischerweise L-Oligonukleotide, die von natürlichen Enzymen infolge ihres Aufbaus aus L-Nukleotiden praktisch nicht abgebaut werden können. Neben der Zielmolekül-Spezifität qualifiziert sie diese Eigenschaft zur Anwendung in den verschiedensten Bereichen wie bspw. Analyse biologischer Probe, Diagnose und Therapie.

Ähnlich wie bei anderen chemischen Verbindungen, die in der Diagnostik sowie Therapie Anwendung finden, insbesondere solche, die in einem Organismus appliziert werden, besteht somit auch für Spiegelmere ein Bedarf, diese in eine Form zu überführen, welche erlaubt, dass die Spiegelmere über einen längeren Zeitraum in einem Organismus vorhanden und wirksam sind. Dabei ist es eine weitere der vorliegenden Erfindung zugrunde liegenden Aufgabe, dass durch die Modifikation die für die Spiegelmere charakteristische Ziel-Molekül-Spezifität nicht beeinflusst wird.

Erfindungsgemäß wird die Aufgabe in einem ersten Aspekt gelöst durch eine L-Nukleinsäure umfassend einen L-Nukleinsäure-Teil und einen Nicht-L-Nukleinsäure-Teil, wobei die Konjugation mit dem Nicht-L-Nukleinsäure-Teil zu einer verlangsamten Ausscheidung aus einem Organismus bzw. renalen Clearance führt, verglichen mit einer L-Nukleinsäure umfassend nur den L-Nukleinsäure-Teil.

In einem zweiten Aspekt wird die der Erfindung zugrunde liegenden Aufgabe gelöst durch eine modifizierte L-Nukleinsäure umfassend einen L-Nukleinsäure-Teil und einen Nicht-L-Nukleinsäure-Teil, wobei die Konjugation mit dem Nicht-L-Nukleinsäureteil zu einer erhöhten Verweildauer in einem Organismus führt, verglichen mit einer L-Nukleinsäure umfassend nur den L-Nukleinsäure-Teil.

In einem dritten Aspekt wird die der Erfindung zugrundeliegende Aufgabe gelöst durch eine modifizierte L-Nukleinsäure, insbesondere eine erfindungsgemäße modifizierte L-Nukleinsäure, umfassend einen L-Nukleinsäure-Teil und einen Nicht-L-Nukleinsäure-Teil, wobei der Nicht-L-Nukleinsäure-Teil ein Molekulargewicht von mehr als etwa 300 Da, bevorzugterweise mehr als etwa 20.000 Da und bevorzugtererweise mehr als etwa 40.000 Da aufweist

In einer Ausführungsform der erfindungsgemäßen modifizierten L-Nukleinsäuren ist vorgesehen, dass der L-Nukleinsäure-Teil mit dem Nicht-L-Nukleinsäure-Teil konjugiert ist und der Nicht-L-Nukleinsäure-Teil ein Molekulargewicht von mehr als etwa 300 Da, bevorzugterweise mehr als etwa 20.000 Da und bevorzugtererweise mehr als etwa 40.000 Da aufweist.

In einer weiteren Ausführungsform der erfindungsgemäßen modifizierten L-Nukleinsäuren ist vorgesehen, dass die modifizierte L-Nukleinsäure ein Molekulargewicht von etwa 600 bis 500,000 Da, bevorzugterweise von etwa 10,000 bis 400,000 Da bevorzugtererweise von etwa 50,000 bis 300,000 Da aufweist.

In einer noch weiteren Ausführungsform der erfindungsgemäßen modifizierten L-Nukleinsäuren ist vorgesehen, dass der L-Nukleinsäure-Teil ein Molekulargewicht von 300 bis 50,000 Da, bevorzugterweise von 5,000 bis 25,000 Da bevorzugtererweise von 7,000 bis 15,000 Da aufweist.

Schließlich ist in einer Ausführungsform der erfindungsgemäßen modifizierten L-Nukleinsäuren vorgesehen, dass der Nicht-L-Nukleinsäure-Teil an den L-Nukleinsäure-Teil über eine funktionale Gruppe des L-Nukleinsäure-Teils direkt oder indirekt verknüpft ist, die an einem der folgenden Bestandteile der L-Nukleinsäure vorhanden oder gebunden ist, wobei die funktionale Gruppe ausgewählt ist aus der Gruppe, die terminale und nicht-terminale Phosphate, terminale und nicht-terminale Zuckeranteile und natürliche und nicht-natürliche Purin- und natürliche und nicht-natürliche Pyrimidin-Basen umfasst.

In einer Ausführungsform der erfindungsgemäßen modifizierten L-Nukleinsäuren ist vorgesehen, dass die Verknüpfung des Nicht-L-Nukleinsäure-Teils mit dem L-Nukleinsäure-Teil über die 2'-OH-, 3'-OH- und/oder 5'-OH-Gruppe oder eines Derivates davon eines oder mehrerer der Zuckeranteile des L-Nukleinsäure-Teils vorliegt.

In einer weiteren Ausführungsform der erfindungsgemäßen modifizierten L-Nukleinsäuren ist vorgesehen, dass die Verknüpfung über mindestens eine der Positionen 5 oder 6 der Pyrimidin-Base vorliegt.

In einer noch weiteren Ausführungsform der erfindungsgemäßen modifizierten L-Nukleinsäuren ist vorgesehen, dass die Verknüpfung über mindestens einer der Position 8 der Purin-Basen vorliegt.

Schließlich ist in einer Ausführungsform der erfindungsgemäßen modifizierten L-Nukleinsäuren vorgesehen, dass die Verknüpfung an einer oder mehreren der exozyklischen und/oder endozyklischen Amingruppen und/oder Ketogruppen der Purin- und/oder Pyrimidin-Basen und/oder abasischen Position(en) vorliegt.

In einer Ausführungsform der erfindungsgemäßen modifizierten L-Nukleinsäuren ist vorgesehen, dass der Nicht-Nukleinsäure-Teil ausgewählt ist aus der Gruppe, die lineares Poly(ethylen)glykol, verzweigtes Poly(ethylen)glykol, Hydroxyethylstärke, Peptide, Proteine, Polysaccharide, Sterole, Polyoxypropylen, Polyoxyamidate, Poly(2-hydroxyethyl)-Lglutamin, precise Polyethylenglykol umfasst.

In einer Ausführungsform der erfindungsgemäßen modifizierten L-Nukleinsäuren ist vorgesehen, dass zwischen dem L-Nukleinsäure-Teil und dem Nicht-L-Nukleinsäure-Teil ein Linker angeordnet ist.

In einer weiteren Ausführungsform der erfindungsgemäßen modifizierten L-Nukleinsäuren ist vorgesehen, dass der L-Nukleinsäure-Teil eine Nukleinsäure umfasst gemäß SEQ ID NO. 1.

In einer bevorzugten Ausführungsform der erfindungsgemäßen modifizierten L-Nukleinsäuren ist vorgesehen, dass der L-Nukleinsäure-Teil am 5'-OH-Ende als Linker 6-Aminohexylphosphat aufweist.

In einer besonders bevorzugten Ausführungsform der erfindungsgemäßen modifizierten L-Nukleinsäuren ist vorgesehen, dass an das freie Amin des Aminohexylphosphat-Linkers Polyethylenglykol gekoppelt ist.

In einem vierten Aspekt wird die Aufgabe gelöst durch die Verwendung der erfindungsgemäßen L-Nukleinsäuren als Diagnostikum oder diagnostisches Mittel.

In einem fünften Aspekt wird die der Erfindung zugrunde liegenden Aufgabe gelöst durch die Verwendung der erfindungsgemäßen modifizierten L-Nukleinsäuren zur Herstellung eines Medikaments.

In einem sechsten Aspekt wird die Aufgabe gelöst durch ein Verfahren zur Bereitstellung einer modifizierte L-Nukleinsäure, insbesondere der erfindungsgemäßen Nukleinsäuren, umfassend einen L-Nukleinsäure-Teil und einen Nicht-L-Nukleinsäure-Teil, wobei die folgenden Schritte vorgesehen sind:
a) Bereitstellen einer L-Nukleinsäure, welche dem L-Nukleinsäure-Teil oder einem Teil davon der modifizierte. L-Nukleinsäure ausbildet;
b) Bereitstellen einer Nicht-L-Nukleinsäure, welche den Nicht-L-Nukleinsäure-Teil oder einen Teil davon der modifizierte L-Nukleinsäure ausbildet; und
c) Umsetzen der L-Nukleinsäure aus a) und der Nicht-L-Nukleinsäure aus b), und
d) optional Isolieren der in Schritt c) erhaltenen modifizierten L-Nukleinsäure.

In einer Ausführungsform des erfindungsgemäßen Verfahrens ist vorgesehen, dass die L-Nukleinsäure in Schritt a) einen Linker umfasst.

In einer weiteren Ausführungsform des erfindungsgemäßen Verfahrens ist vorgesehen, dass nach dem Bereitstellen der L-Nukleinsäure in Schritt a) diese mit einem Linker versehen wird.

Es ist weiterhin im Rahmen der vorliegenden Erfindung, dass der Nicht-L-Nukleinsäure-Teil einen Linker umfasst bzw. das nach dem Bereitstellen der Nicht-L-Nukleinsäure in Schritt b) diese mit einem Linker versehen wird.

Der vorliegenden Erfindung liegt die überraschende Erkenntnis zugrunde, dass bei der Verwendung von L-Nukleinsäuren in einem Organismus wie bspw. einem Säugetierorganismus und ganz besonders bei einem Säugetier das bevorzugterweise ausgewählt ist aus der Gruppe, die Menschen, Affen, Hunde, Katzen, Schafe, Ziegen, Kaninchen, Meerschweine, Mäuse und Ratten umfasst, zwar L-Nukleinsäuren nicht metabolisiert werden, was darin begründet liegt, dass die in derartigen Organismen in der Regel vorkommenden Nukleasen L-Nukleinsäuren aufgrund ihrer Stereospezifität nicht als Substrat erkennen, die biologische Halbwertszeit der L-Nukleinsäuren im besagten Organismus nichtsdestotrotz vergleichsweise gering ist. So wurde festgestellt, dass bei in-vivo Verabreichung von unmodifizierten L-Nukleinsäuren mit einer Zufallsequenz in Ratten und Affen die Halbwertszeit zwischen 30 Minuten und 6 Stunden betrug. Auch bei der Verwendung einer L-Nukleinsäure, d.h. eines Spiegelmers, die gegen ein in dem untersuchten Organismus vorhandenes Zielmolekül gerichtet war, bestätigte sich die vorstehende Beobachtung einer vergleichsweise kurzen biologischen Halbwertszeit, was bestätigt, dass diese sich nicht auf einem Artefact infolge der nicht-Spezifität der L-Nukleinsäure gründet. In dem konkret vorstehend beschriebenen Fall handelt es sich um ein Spiegelmer für den Hormon-Agonisten Gonadotropin-freisetzendes Hormon (GnRH), welches männlichen, orchidektomierten Ratten verabreicht wurde. GnRH stimuliert die Synthese und Freisetzung der Gonadotropine Follikel-stimulierendes Hormon (FSH) und luteinisierendes Hormon (LH). Bei männlichen, orchidektomierten Ratten liegen aufgrund des fehlenden Testosteron Feedback-Signals erhöhte FSH- und LH-Spiegel vor. Das spezifische Spiegelmer bedingte eine deutliche Absenkung des LH-Spiegels in einer ersten Studie (100 mg/kg, s.c. Applikation), allerdings konnte bereits nach wenigen Stunden eine Abnahme der Effektivität des Spiegelmers beobachtet werden. In einer Durchführung derselben Studie mit dem korrespondierenden GnRH-Spiegelmer-PEG-Konjugat (150 mg/kg, i.v. Applikation) konnte hingegen eine vollständige Absenkung des LH-Spiegels beobachtet werden, der über einen Zeitraum von 24 Stunden nach wie vor vollständig abgesenkt blieb. Das GnRH-Spiegelmer-PEG-Konjugat stellt ein Beispiel für eine erfindungsgemäße modifizierte L-Nukleinsäure dar. Das GnRH-Spiegelmer entspricht dabei dem L-Nukleinsäure-Teil und das PEG dem Nicht-L-Nukleinsäure-Teil.

Diese Ergebnisse zeigen, dass die Verweildauer von L-Nukleinsäuren wie Spiegelmeren in einem Organismus durch eine Modifikation, insbesondere eine hoch-molekulare Modifikation der L-Nukleinsäure verlängert werden kann. Die Modifikation der L-Nukleinsäure erfolgt durch Verknüpfen derselben mit einer Nicht-L-Nukleinsäure. Ohne darauf festgelegt sein zu wollen, scheint die überraschende Beobachtung darin begründet zu liegen, dass infolge des erhöhten Molekulargewichts der solchermaßen modifizierten L-Nukleinsäure und der fehlenden Metabolisierbarkeit der L-Nukleinsäure die Ausscheidung der modifizierten L-Nukleinsäure aus einem Organismus, insbesondere einem Säugetierorganismus, verlangsamt wird. Da die Ausscheidung typischerweise über die Nieren erfolgt, wird derzeit davon ausgegangen, dass die glomeruläre Filtrationsrate der Nieren hinsichtlich der modifizierten L-Nukleinsäuren gegenüber derjenigen der nicht-modifizierten L-Nukleinsäuren signifikant verringert ist, was zu einer erhöhten Verweildauer, d. h. biologischen Halbwertszeit der modifizierten L-Nukleinsäure verglichen mit der Verweildauer der korrespondierenden, aber nicht-modifizierten L-Nukleinsäure führt.

Besonders beachtlich ist in diesem Zusammenhang die Tatsache, dass trotz der vorgenommenen Modifikation die modifizierte L-Nukleinsäure, d.h. insbesondere der für die Ziel-Molekül-Spezifität verantwortliche L-Nukleinsäure-Teil davon, an ihrer Spezifität offensichtlich nichts einbüßt. Damit weisen die erfindungsgemäßen modifizierten L-Nukleinsäuren in vollkommen überraschender Weise jene Eigenschaften auf, wie man sie ansonsten bei anderen pharmazeutisch aktiven Verbindungen normalerweise nicht realisieren kann, dass nämlich auf umfangreiche galenische Formulierungen, bspw. in Form von Depotpräparaten, die sukzessive den Wirkstoff abgeben, verzichtet und vielmehr eine direkte Modifizierung des in Frage stehenden Wirkstoffes herbeigeführt werden kann, ohne dass dabei dessen biologische Aktivität, im Falle der Spiegelmere insbesondere ausgedrückt als Spezifität der Reaktion oder Komplexbildung mit ihrem jeweiligen Zielmolekül, nachteilig beeinflusst wird. Mit anderen Worten, die erfindungsgemäßen modifizierten L-Nukleinsäuren überwinden die ansonsten bei pharmazeutisch aktiven Wirkstoffen und insbesondere bei kleinen Wirkstoffmolekülen existierende Unvereinbarkeit von spezifischer Aktivität des pharmazeutischen Wirkstoffes mit einer Erhöhung seiner Verweildauer in einem Organismus, insbesondere mit einer Verringerung der Ausscheidung wie bspw. der glomerulären Filtrationsrate. Dabei ist beachtlich, dass die Affinität des L-Nukleinsäure-Teils durch die Konjugation mit dem Nicht-L-Nukleinsäure-Teil im wesentlichen unverändert bleibt.

Das vorstehend Gesagte gilt selbstverständlich nicht nur für den Fall der Anwendung modifizierter L-Nukleinsäuren wie Spiegelmere als therapeutisch wirksame Agenzien, sondern auch für deren Verwendung als diagnostische Mittel, insbesondere bei deren Verwendung als in-vivo Diagnostika. Ein typisches Beispiel der Verwendung von Spiegelmeren als in-vivo Diagnostika stellt das in-vivo Imaging dar und hierbei insbesondere die Verwendung von Radionuklid tragenden Spiegelmeren für die Positronen-Emissionstomographie. Bei dieser Anwendung wird darauf abgestellt, dass das Radionuklid für einen genau definierten Zeitrahmen im Körper erhalten bleibt. Würde das Radionuklid und damit die Radioaktivität über einen längeren Zeitraum im Organismus verbleiben, wobei länger so zu verstehen ist, wie erforderlich, um die jeweilige Untersuchung durchzuführen, würde dies mit einer für den Patienten unnötigen, in einigen Fällen möglicherweise sogar gesundheitsgefährdenden Exposition mit radioaktiver Strahlung einhergehen. Andererseits würde für den Fall, dass die Ausscheidung des diagnostischen Mittels und damit der radioaktiven Markierung aus dem Körper zu schnell erfolgt, dazu führen, dass keine geeignete Diagnose oder diagnostische Aussage möglich wäre. Mit der Verfügbarkeit der erfindungsgemäßen modifizierten L-Nukleinsäuren kann in Abhängigkeit von den jeweiligen Erfordernissen das diagnostische Mittel hinsichtlich seiner Verweildauer, d. h. seiner biologischen Halbwertszeit, optimiert eingestellt werden. Dies gründet sich maßgeblich mit auf der Beobachtung, dass die glomeruläre Filtrationsrate ab einem Molekulargewicht von größer als etwa 45.000 Da stark eingeschränkt wird. Ansonsten ist die Ausscheidung, insbesondere die glomeruläre Filtrationsrate eindeutig mit der Molekülgröße korreliert. Durch die Verwendung eines geeigneten Nicht-L-Nukleinsäure-Teils wie beispielsweise den hierin beschriebenen kann die Verweildauer der modifizierten L-Nukleinsäure exakt auf die Erfordernisse angepasst werden.

Die chemisch Natur des Nicht-L-Nukleinsäure-Teils der modifizierten L-Nukleinsäure kann im Rahmen bestimmter Grenzen praktisch frei gestaltet werden. Eine Anforderung an die modifizierte Nukleinsäure, die einem Organismus verabreicht wird, die in der Vielzahl der Anwendungsfälle der modifizierten L-Nukleinsäure gegeben sein sollte, besteht darin, dass der Nicht-L-Nukleinsäure-Teil aus einer oder mehreren nicht-immunogenen Verbindungen besteht. Alternativ, aber ggf. auch ergänzend hierzu, kann es sich dabei um lipophile Verbindungen ebenso wie um hydrophile Verbindungen handeln. Es ist für den Fachmann offensichtlich, dass in Abhängigkeit von den Rahmenbedingungen des Einzelfalles möglicherweise auch leicht immunogene Verbindungen herangezogen werden können, insbesondere dann, wenn die Verabreichung der erfindungsgemäßen modifizierten L-Nukleinsäure nicht über einen längeren Zeitraum oder mehrfach erfolgen soll, sondern lediglich einmalig. Umgekehrt ist dieser Aspekt besonders dann von Bedeutung, wenn die erfindungsgemäßen modifizierten L-Nukleinsäuren über einen längeren Zeitraum oder mehrfach verabreicht werden müssen. Dabei wird in der Regel sicherzustellen sein, dass durch den Nicht-L-Nukleinsäure-Teil der modifizierte L-Nukleinsäure keine Immunantwort bei Applikation der modifizierten L-Nukleinsäure erzeugt wird, die bei erneuter Verabreichung derselben zu einer immunologischen oder allergischen Reaktion führen würde.

Die Ausgestaltung des Nicht-L-Nukleinsäure-Teils der modifizierte L-Nukleinsäure kann dabei auch so erfolgen, dass mehr als ein Nicht-L-Nukleinsäure-Teil an einen L-Nukleinsäure-Teil gebunden oder konjugiert wird. Beispielsweise ist es möglich, dass zwei oder mehrere Nicht-L-Nukleinsäure-Teile an den L-Nukleinsäure-Teil gebunden werden. Der einzelne Nicht-L-Nukleinsäure-Teil ist bevorzugterweise ein Polymer, wobei die Untereinheiten des Polymers ein vergleichsweise geringes Molekulargewicht aufweisen können. Es ist auch im Rahmen der vorliegenden Erfindung, dass mehr als ein L-Nukleinsäure-Teil an einen Nicht-L-Nukleinsäure-Teil gebunden ist.

Ein weiterer Aspekt, der bei der Auswahl des Nicht-L-Nukleinsäure-Teils der modifizierte L-Nukleinsäure zu berücksichtigen ist, besteht in dem Adressieren der Spiegelmere an bestimmte Verbindungen, insbesondere an bestimmte Organe oder Zellen. Hier kann in Abhängigkeit von den spezifischen Verhältnissen der Nicht-L-Nukleinsäure-Teil so angepasst werden, dass sich, unabhängig von der durch den L-Nukleinsäure-Teil bedingten Spezifität der Bindung des Spiegelmers oder der modifizierten L-Nukleinsäure, die modifizierte L-Nukleinsäure bevorzugt in bestimmten Zellen, Geweben oder Organen anreichert.

Typischerweise beträgt das Molekulargewicht des Nicht-L-Nukleinsäure-Teils zwischen 300 und 500.000 Da. Der L-Nukleinsäure-Teil kann entweder einzeln, mehrfach, oder in allen beliebigen Kombinationen mit anderen Nicht-L-Nukleinsäure-Teilen an gleiche oder verschiedene Stellen des L-Nukleinsäure-Teils der modifizierte L-Nukleinsäure gekoppelt werden.

Es ist im Rahmen der vorliegenden Erfindung, dass das Molekulargewicht der modifizierten L-Nukleinsäure stark von dem Nicht-L-Nukleinsäure-Teil bestimmt wird. Grundsätzlich kann die modifizierte L-Nukleinsäure ein Molekulargewicht von etwa 600 bis 500.000 Da aufweisen, bevorzugterweise von etwa 10.000 bis 400.000 Da und bevorzugtererweise von etwa 50.000 bis 300.000 Da. Geringere Molekulargewichtebereiche werden beispielsweise durch solche modifizierten L-Nukleinsäuren realisiert, die Chlosterol-Konjugate sind und typischerweise ein Molekulargewicht von etwa 10 kDa bis 25 kDa aufweisen. Höhere Molekulargewichtsbereiche werden beispielsweise durch solche modifizierten L-Nukleinsäuren realisiert, die HES-Konjugate sind und oftmals ein Molekulargewicht von etwa 100 kDa bis 500 kDa aufweisen. Für den Fall, dass die modifizierten L-Nukleinsäuren PEG-Konjugate sind, ist das bevorzugte Molekulargewicht etwa 40 bis 70 kDa.

Als Nicht-L-Nukleinsäure-Teil kann beispielsweise verwendet werden:
- Polyether und Alkoxypolyether, wie z.B. lineare oder verzweigte Poly(ethylen)glykole (PEG), Methoxypoly(ethylen)glykole, Ethoxypoly(ethylen)glykole, precise PEG (wobei precise PEG ein Polyamid der Form (-NH-Y-NH-CO-X-CO-) ist, wobei Y und X an jeder Stelle variiert werden können als (-CH₂CH₂O-)ₚ mit unterschiedlichen p im Bereich von 4-6), Poly(2-hydroxyethyl)-L-glutamine und Polyoxypropylene, die sich insbesondere dadurch auszeichnen, dass sie ihrerseits nicht in vivo metabolisierbar sind und insoweit der durch die Größe, d.h. das Molekulargewicht der modifizierten L-Nukleinsäure bedingte Effekt der kontrollierten Ausscheidung besonders nachhaltig ausgeprägt ist und nicht durch Abbauprozesse an dem Nicht-L-Nukleinsäure-Teil überlagert werden.
- Peptide, Polypeptide und Proteine wie z.B. Albumin, wobei es sich bei diesen Verbindungen sowohl um natürlicherweise vorhandene als auch um von außen hinzugefügte Substanzen handeln kann.
- Polysaccharide, wie z.B. Hydroxyethylstärke, Dextrane, sind ihrerseits metabolisierbar und können infolge der genau kontrollierbaren Abbaurate auch die Verweildauer sehr spezifisch beeinflussen. Das Molekulargewicht der in einer Ausführungsform verwendeten Hydroxyethylstärke liegt zwischen 10 kDa, bevorzugterweise zwischen 40 kDa und 400 kDa, bevorzugterweise zwischen 100 kDa und 300 kDa. Hydroxyethylstärke hat einen molaren Substitutionsgrad von 0.1 bis 0.8 und ein Verhältnis von C₂ : C₆ im Bereich von 2 bis 20. Hinsichtlich der Kopplung der Polysaccharide an den L-Nukleinsäure-Teil der modifizierten L-Nukleinsäure gilt das hierin im Zusammenhang mit dem Zuckeranteil der L-Nukleinsäuren hinsichtlich der Verwendung der OH-Gruppen und deren Derivatisierung Gesagte.
- Sterole, wie z.B. Cholesterin. Zwar zeichnen sich Sterole durch ein vergleichsweise geringes Molekulargewicht aus, jedoch kann auch dieses bereits zu einer Erhöhung der Verweilzeit der damit modifizierten L-Nukleinsäure führen. Von größerer Bedeutung in diesem Zusammenhang ist jedoch das Verhalten der Sterole, insbesondere von Cholesterin zu bewerten, das in vivo einen nicht kovalenten Komplex mit Lipoproteinen wie zum Beispiel HDL bildet, wodurch eine Molekülvergrößerung und damit eine längere Halbwertszeit in vivo erreicht wird.

Grundsätzlich ist es im Rahmen der vorliegenden Erfindung, dass der Nicht-L-Nukleinsäureteil auch von einem oder mehreren D-Nukleosiden bzw. D-Nukleotiden ausgebildet wird, wobei diese einzeln oder insgesamt weitere Modifizierungen aufweisen können, wie beispielsweise Modifizierungen zur Erhöhung der Stabilität in biologischen Systemen. Derartige Modifizierungen stellt beispielsweise die Fluorierung an der Position 2' des Zuckerbestandteiles der Nukleotide bzw. Nukleoside dar. Weiterhin können diese D-Nukleoside und D-Nukleotide Bestandteil der verschiedenen Nicht-L-Nukleinsäuren darstellen, insbesondere derjenigen der vorstehend genannten, aber auch Teil eines der hierin beschriebenen Linkers. Dabei ist es im Rahmen der vorliegenden Erfindung, dass einzelne oder mehrere der D-Nukleoside oder D-Nukleotide auch eine oder mehrere abasische Positionen umfassen können.

Die Verknüpfung des L-Nukleinsäure-Teils mit einem oder mehreren der Nicht-L-Nukleinsäure-Teile kann grundsätzlich an allen Bestandteilen oder Gruppierungen der beiden die modifizierte L-Nukleinsäure aufbauenden Teile erfolgen, wobei auch vorgesehen sein kann, dass Derivatisierungen an einer oder mehreren Stellen eines oder beider Teile, d.h. an der L-Nukleinsäure ebenso wie dem/den Nicht-L-Nukleinsäure-Teil(en), erfolgen. Die Verknüpfung kann insbesondere an der 5'-OH-, 3'-OH- oder 2'-OH-Gruppe der L-Nukleinsäure, insbesondere des Ribose- oder Desoxyribose-Teils davon, erfolgen.

Dabei ist es auch im Rahmen der vorliegenden Erfindung, dass zumindest ein Teil der Zuckerbestandteile der die L-Nukleinsäure aufbauenden Nukleotide einen anderen Zucker als Ribose oder Deoxyribose aufweisen kann. Derartige Zucker können beispielsweise weitere Pentosen wie zum Beispiel Arabinose sein aber auch Hexosen oder Tetrosen oder auch ein Stickstoffatom enthalten wie zum Beispiel in einem Morpholino-Ring oder Aza- oder Thiozucker oder weitere Zuckermodifikationen wie in locked Nukleinsäuren (LNA) oder Peptid Nukleinsäuren (PNA) Diese OH-Gruppen können durch geeignete chemische Modifizierung vorliegen als NH₂-, SH-, Aldehyd-, Carboxysäure-, Phosphat-, Jod-, Brom-, oder Chlorgruppen. Dem Fachmann sind weitere funktionale Gruppen, die eine Kopplung an den L-Nukleinsäure-Teil erlauben, bekannt. Sofern hierin die Verknüpfung eines Nicht-L-Nukleinsäure-Teils mit einer L-Nukleinsäure beschrieben wird, gelten die Ausführungen sinngemäß auch für den Fall, dass mehr als ein Nicht-L-Nukleinsäure-Teil an den L-Nukleinsäure-Teil gebunden wird oder daran gebunden vorliegt, sofern keine gegenteiligen Aussagen getroffen werden.

Es ist weiter im Rahmen der vorliegenden Erfindung, dass zumindest ein Teil der Phosphatgruppen der die modifizierte L-Nukleinsäure aufbauenden Nukleotide Modifikationen aufweisen. Derartige Modifikationen sind beispielsweise Phosphothioate, Phosphodithioate, Phosphoramidate, Phosphonate und weitere den Fachleuten bekannte Modifikationen.

Neben der Verknüpfung des L-Nukleinsäure-Teils an den Nicht-L-Nukleinsäure-Teils über den Zucker-Teil des L-Nukleinsäure-Teils kann die Verknüpfung ebenso am Phosphat-Rückgrat erfolgen, wobei auch hier wie im Zusammenhang mit der Verknüpfung über den Ribose- oder Deoxyribose-Anteil der L-Nukleinsäure angegeben, eine entsprechende Modifizierung erfolgen kann. Schließlich sind Verknüpfungen über die Position 5 und/oder 6 der Pyrimidinbase(n), Position 8 der Purinbase(n) sowie die exo- und endozyklischen Aminund Ketogruppen der jeweiligen Nukleobasen möglich, ggf. auch nach funktioneller Modifikation derselben, wie vorstehend ausgeführt. Neben natürlichen Basen kann die L-Nukleinsäure auch eine oder mehrere nicht-natürliche Basen enthalten, wie z.B. Isoguanidin, Isocytidin, Xanthosin, Inosin, 2,4-Diaminopyrimidin. Dabei ist es im Rahmen der vorliegenden Erfindung, dass eine jegliche der hierin beschriebenen Verknüpfungen zwischen dem L-Nukleinsäure-Teil und dem Nicht-Nukleinsäure-Teil direkt oder indirekt erfolgen kann. Eine indirekte Verknüpfung liegt insbesondere dann vor, wenn zwischen dem L-Nukleinsäure-Teil und dem Nicht-L-Nukleinsäure-Teil ein Linker, beispielsweise ein hierin beschriebener Linker angeordnet ist und dieser eine oder beide der funktionalen Gruppen zur Verfügung stellt.

Es ist auch im Rahmen der vorliegenden Erfindung, dass zwischen dem L-Nukleinsäure-Teil und dem Nicht-L-Nukleinsäure-Teil(en) ein oder mehrere sogenannter Linker eingebaut werden kann. Ein derartiger Linker besteht typischerweise aus mindestens einer funktionalen Gruppe sowie einem Abstandshalter oder Spacer. Die Funktion des Linkers kann zum einen darin bestehen, die Kopplungsreaktion zu erleichtern. In Ergänzung oder alternativ dazu kann ihm eine Funktion dergestalt zukommen, dass eine räumliche Distanz zwischen dem L-Nukleinsäure-Teil und dem Nicht-L-Nukleinsäure-Teil der modifizierte L-Nukleinsäure aufgebaut wird. Eine derartige Distanz ist unter bestimmten Bedingungen von Vorteil, bspw. dann, wenn Wechselwirkungen zwischen den die modifizierte L-Nukleinsäure aufbauenden Teilen, insbesondere zwischen dem L-Nukleinsäure-Teil und dem Nicht-L-Nukleinsäure-Teil oder zwischen zwei oder mehreren Nicht-L-Nukleinsäure-Teilen der modifizierten L-Nukleinsäure verhindert werden sollen.

Der Linker selbst kann wiederum eine oder mehrere funktionelle Gruppen umfassen und an einer der oben genannten Stellen des L-Nukleinsäure-Teils an diesen gekoppelt sein. Typischerweise besteht der Spacer, u.a. aus unterschiedlich langen Alkylketten, wobei eine Kettenlänge von 1 bis 20, insbesondere 4 bis 15 und ganz besonders 6 bis 12 C-Atomen bevorzugt ist. Die Alkylkette kann selbst verzweigt sein oder weitere funktionelle Gruppen tragen. Eine typische Ausführungsform des Spacers umfasst Etherverknüpfungen zwischen einzelnen Monomeren wie diese bspw. bei Poly(ethylen)glykol oder Polyoxypropylen vorhanden sind, wobei hier oftmals die einzelnen Monomeren in den Polymeren 1- bis 20-mal vorhanden sind. Auch bei der Ausbildung des Spacers durch Polyaminalkyl- oder Polyamidoalkyl-Ketten ist eine Häufigkeit der diese Polymeren aufbauenden Monomeren mit einem Wert von 1 bis 20 üblich.

Der Linker kann entweder an eines der L-Nukleotide gekoppelt sein, die den L-Nukleinsäure-Teil der modifizierte L-Nukleinsäure ausbilden. Alternativ kann der Linker auch während der enzymatischen oder chemischen Synthese der L-Nukleinsäure in das entstehende Oligomer eingebaut werden. Desweiteren ist es im Rahmen der vorliegenden Erfindung, dass die L-Nukleinsäure post-synthetisch modifiziert und dadurch mit einem Linker für die Kopplung eines Nicht-L-Nukleinsäure-Teils versehen wird.

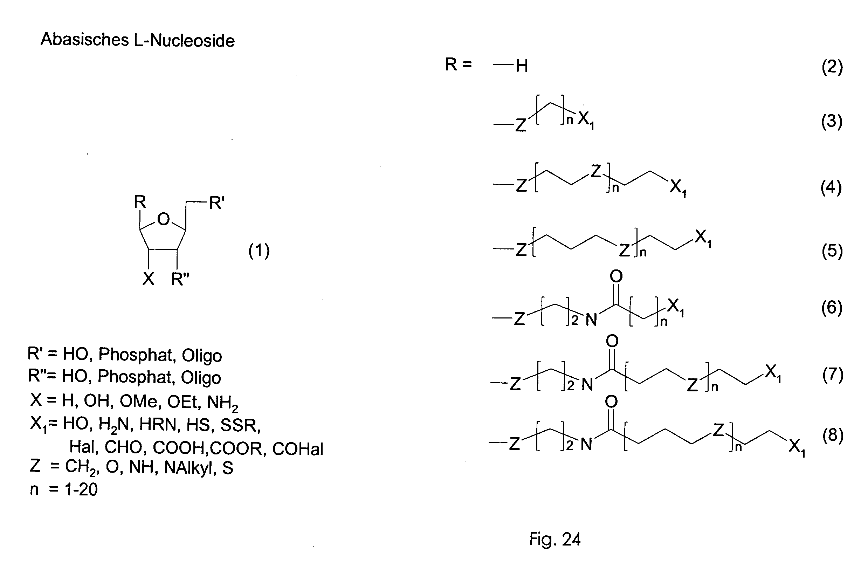

Es ist auch im Rahmen der vorliegenden Erfindung, dass ein Linker zum Beispiel an eine abasische Position in einem Hairpin-Loop oder am 3'- oder 5'-Ende oder einer anderen Position, eingebaut wird. Handelt es sich bei der L-Nukleinsäure um ein Spiegelmer kann die abasische Position an einer Position des Spiegelmers eingebaut sein, die nicht für die Bindung des Zielmoleküls bzw. für die Struktur des Spiegelmers essentiell wichtig ist. Mit abasischer Position soll hierin eine Stelle des L-Nukleinsäure-Teils bezeichnet werden, die analog zu einem normalen L-Nukleotid das gleiche Rückgrat aus Phosphat und Zucker besitzt, bei der die Nukleobase jedoch durch ein Wasserstoffatom oder einen Linker substituiert ist, wie dies auch in Fig. 24 dargestellt ist.

Die modifizierten L-Nukleinsäuren, die hierin auch als L-Nukleinsäure-Konjugate bezeichnet werden, können, wie bereits aus der oben angegebenen Aufstellung hinsichtlich der Verknüpfungsorte zwischen dem L-Nukleinsäure-Teil und dem Nicht-L-Nukleinsäure-Teil ersichtlich, durch eine Vielzahl von Reaktionen hergestellt werden, wie im folgenden detaillierter ausgeführt wird
- Säureamide können hergestellt werden durch Umsetzung von N-Hydroxysuccinimid (NHS) oder ähnlichen Aktivierungen von Carbonsäuren, wie Anhydrid, Säurechlorid, Ester, Succinimid und Maleimid mit einem primären oder sekundären Amin ^{1,2}.
- Thioether können ausgehend von einem Halogenid und einem Thiol hergestellt werden ^{3,4} und anschließend Gegenstand einer Oxidation zu Sulfoxid oder Sulfon sein ^{5,6}. Halogenide, insbesondere Haloacetyl (Iodessigsäure, Bromessigsäure), können an eine beliebige funktionale Gruppe eines der beiden L-Nukleinsäure-Teile per Ester- oder Säureamidbindung gekoppelt werden und anschließend die sehr reaktive Iod- oder Bromgruppe mit einem freien Thiol gekoppelt werden. Haloacetyl stellt somit einen Spezialfall der Halogenid-Thiol-Kopplung dar. ⁷. Thioether können außerdem ausgehend von Maleimid und Thiol hergestellt werden ⁷⁻⁹, Isothioharnstoff aus Isothiocyanat und Amin ¹⁰, Isoharnstoff ausgehend von Isocyanat und Amin ¹¹, Carbamat ausgehend von Isocyanat und Alkohol ¹², C-C-Verknüpfungen mittels Diels-Alder-Reaktion ¹³, Heterocyclen durch 1,3-dipolare Cycloaddition ¹³, Amine durch reduktive Aminierung, im Anschluss an die Umsetzung von beispielsweise Aldehyd oder Keton mit einem Amin unter anschließender Reduktion ¹⁴, Säureamid ausgehend von einer Säure und einem Amin ^{15,16}, Ester ausgehend von Carbonsäure oder den oben erwähnten aktivierten Carbonsäuren und Alkohol, Sulfonamid ausgehend von Amin und Sulfonylchlorid ¹⁷, sekundäre Amine ausgehend von einem Epoxid und einem Amin ^{18,19}, Thioether ausgehend von einem Epoxid und einem Thiol ²⁰, ein Disulfid ausgehend von einem Thiol und einem weiteren Thiol oder einem Disulfid ^{21,22}, Hydrazone ausgehend von einem Hydrazin und einem Aldehyd oder einem Keton, wobei das Hydrazon weiter zu einem stabilen modifizierten Hydrazin reduziert werden kann ²³, Phosphothioate ausgehend von einem Phosphat oder einer aktivierten Phosphorsäure wie beispielsweise Phosphoroimidazolid und einem Thiol ²⁴, Phosphoramidat ausgehend von einem Phosphat oder einer aktivierten Phosphorsäure wie beispielsweise Phosphoroimidazolid oder Phos-N-Hydroxydbenzotriazol und einem Amin ²⁴⁻²⁷. Dabei ist es im Rahmen der vorliegenden Erfindung ist, zuerst einen Linker über eine Phosphoramidatoder Phosphothioat-Verknüpfung an den L-Nukleinsäure-Teil zu koppeln und anschließend an den Nicht-L-Nukleinsäure-Teil. Derartige Linker können insbesondere Ethylendiamin oder Cysteamin sein.

Die vorstehenden Ausführungen gelten grundsätzlich sowohl für den Fall, dass die erstgenannte reaktive Ausgangsgruppe am Nicht-L-Nukleinsäure-Teil angeordnet ist, wie für den Fall, dass diese am L-Nukleinsäure-Teil angeordnet ist. Die entsprechende Modifikation der L-Nukleinsäure in dem Sinne, dass eine entsprechende reaktive Gruppe bereitgestellt wird, ist den Fachleuten auf diesem Gebiet bekannt. Gleiches gilt für den Nicht-L-Nukleinsäure-Teil.

Der Begriff L-Nukleinsäure wird hierin synonym zu dem Begriff L-Oligonukleotide oder L-Polynukleotide verwendet und bezeichnet, unter anderem, sowohl L-Desoxyribonukleinsäure wie auch L-Ribonukleinsäure und Kombinationen davon, d. h. dass einzelne oder eine Gruppe von Nukleotiden als RNA vorliegt und die weiteren die Nukleinsäure aufbauenden Nukleotide als DNA vorliegen und umgekehrt. Dabei ist auch vorgesehen, dass anstelle von Desoxyribose oder Ribose andere Zucker die Zuckerkomponente des Nukleotids ausbilden. Weiterhin umfasst ist die Verwendung von Nukleotiden mit weiteren Modifikationen an Position 2' wie NH₂, OMe, OEt, OAlkyl, NHAlkyl und die Verwendung von natürlichen oder unnatürlichen Nukleobasen wie zum Beispiel Isocytidin, Isoguanosin. Es ist dabei auch im Rahmen der vorliegenden Erfindung, dass die L-Nukleinsäure sogenannte abasische Positionen aufweist, d. h. Nukleotide, denen die Nukleobase fehlt. Derartige abasische Positionen können sowohl innerhalb der Nukleotidsequenz der L-Nukleinsäure angeordnet sein, wie auch an einem oder beiden der Enden, d. h. dem 5'- und/oder dem 3'- Ende.

Weiter ist es im Rahmen der vorliegenden Erfindung, dass die L-Nukleinsäure ein oder mehrere D-Nukleoside oder D-Nukleotide enthält. Dabei kann das eine oder die mehreren D-Nukleoside oder Nukleotide sowohl innerhalb der L-Nukleinsäure angeordnet sein, wie auch an einem oder beiden der Enden der L-Nukleinsäure. Das einzelne D-Nukleosid oder D-Nukleotid kann dabei eine oder mehrere Modifizierungen tragen, beispielsweise zur Erhöhung der Stabilität des Nukleosids bzw. Nuklotids bzw. dessen Bindung an die L-Nukleinsäure.

Die L-Nukleinsäure kann grundsätzlich doppel- oder einzelsträngig vorliegen. Typischerweise handelt es sich um eine einzelsträngige L-Nukleinsäure, die jedoch bedingt durch ihre Primärsequenz definierte Sekundärstrukturen und damit auch Tertiärstrukturen ausbilden kann. In der Sekundärstruktur liegen bei einer Vielzahl von L-Nukleinsäuren auch doppelsträngige Abschnitte vor.

Die L-Nukleinsäuren können neben der hierin insbesondere beschriebenen hoch-molekularen Modifizierung auch Modifikationen hinsichtlich der einzelnen Nukleotide der Nukleinsäure bezeichnen, wobei hier z. B. die 2'-OH-Gruppe des Zuckeranteils der Nukleotide als Methylether vorliegen kann, wie bereits vorstehend offenbart.

Bei den hierin beschriebenen markierten L-Nukleinsäuren handelt es sich bevorzugterweise um sog. Spiegelmere. Wie eingangs bereits erwähnt, sind Spiegelmere funktionale Nukleinsäuren, d. h. solche, die an ein Zielmolekül oder einen Teil davon binden, und das Ergebnis des Kontaktierens einer Nukleinsäurebibliothek, insbesondere einer statistischen Nukleinsäurebibliothek, mit dem Zielmolekül sind.

Für ein Selektionsverfahren zur Entwicklung funktionaler Nukleinsäuren werden zunächst kombinatorische DNA-Bibliotheken hergestellt. In der Regel handelt es sich um die Synthese von DNA-Oligonukleotiden, die zentral einen Bereich aus 10 -100 randomisierten Nukleotiden enthalten, die von zwei primer-Bindungsregionen 5'- und 3'-terminal flankiert werden. Die Herstellung derartiger kombinatorischer Bibliotheken ist bspw. beschrieben in Conrad, R.C., Giver, L., Tian, Y. and Ellington, A.D., 1996, Methods Enzymol., Vol 267, 336-367. Eine solche chemisch synthetisierte einzelsträngige DNA-Bibliothek lässt sich über die Polymerase-Kettenreaktion in eine doppelsträngige Bibliothek überführen, die für sich genommen schon für eine Selektion eingesetzt werden kann. In der Regel erfolgt jedoch mit geeigneten Methoden eine Separation der einzelnen Stränge, so dass man wieder zu einer Einzelstrangbibliothek gelangt, die für das in vitro Selektionsverfahren eingesetzt wird, wenn es sich um eine DNA-Selektion handelt (Bock, L.C., Griffin, L.C., Latham, J.A., Vermaas, E.H. und Toole, J.J., 1992, Nature, Vol. 355, 564-566). Es ist jedoch ebenso möglich, die chemisch synthetisierte DNA-Bibliothek direkt in die in vitro Selektion einzusetzen. Darüber hinaus kann prinzipiell aus doppelsträngiger DNA, wenn zuvor ein T7 Promotor eingeführt worden ist, auch über eine geeignete DNA-abhängige Polymerase, z. B. die T7 RNA Polymerase, eine RNA-Bibliothek erzeugt werden. Mit Hilfe der beschriebenen Verfahren ist es möglich, Bibliotheken von 10¹⁵ und mehr DNA- oder RNA-Molekülen zu erzeugen. Jedes Molekül aus dieser Bibliothek hat eine andere Sequenz und somit eine andere dreidimensionale Struktur. Über das in vitro Selektionsverfahren ist es nun möglich, aus der erwähnten Bibliothek durch mehrere Zyklen von Selektion und Amplifikation sowie gegebenenfalls Mutation ein oder mehrere DNA-Moleküle zu isolieren, die gegen ein gegebenes Target eine signifikante Bindungseigenschaft aufweisen. Die Targets können z. B. Viren, Proteine, Peptide, Nukleinsäuren, kleine Moleküle wie Metaboliten des Stoffwechsels, pharmazeutische Wirkstoffe oder deren Metaboliten oder andere chemische, biochemische oder biologische Komponenten sein wie beispielsweise in Gold, L., Polisky, B., Uhlenbeck, O. und Yarus, 1995, Annu. Rev. Biochem. Vol. 64, 763-797 und Lorsch, J.R. und Szostak, J.W., 1996, Combinatorial Libraries, Synthesis, Screening and application potential, ed. Riccardo Cortese, Walter de Gruyter, Berlin, beschrieben. Das Verfahren wird in der Weise durchgeführt, dass bindende DNA- oder RNA-Moleküle aus der ursprünglich eingesetzten Bibliothek isoliert werden und nach dem Selektionsschritt mittels Polymerase-Kettenreaktion amplifiziert werden. Bei RNA-Selektionen ist vor dem Amplifikationsschritt durch Polymerase-Kettenreaktion eine reverse Transkription vorzuschalten. Eine nach einer ersten Selektionsrunde angereicherte Bibliothek kann dann in eine erneute Selektionsrunde eingesetzt werden, so dass die in der ersten Selektionsrunde angereicherten Moleküle die Chance haben, durch Selektion und Amplifikation sich erneut durchzusetzen und mit noch mehr Tochtermolekülen in eine weitere Selektionsrunde zu gehen. Gleichzeitig eröffnet der Schritt der Polymerase-Kettenreaktion die Möglichkeit, neue Mutationen bei der Amplifikation z.B. durch die Variation der Salzkonzentration einzubringen. Nach genügend vielen Selektions- und Amplifikationsrunden haben sich die bindenden Moleküle durchgesetzt. Es ist so ein angereicherter Pool entstanden, dessen Vertreter durch Klonierung vereinzelt und anschließend mit den gängigen Methoden der Sequenzbestimmung von DNA in ihrer Primärstruktur bestimmt werden können. Die erhaltenen Sequenzen werden dann auf ihre Bindungseigenschaften hinsichtlich des Targets überprüft. Das Verfahren zur Erzeugung derartiger Aptamere wird auch als SELEX-Verfahren bezeichnet und ist bspw. beschrieben in EP 0 533 838, dessen Offenbarung hierin durch Bezugnahme aufgenommen wird.

Die besten Bindungsmoleküle können durch Verkürzung der Primärsequenzen auf ihre wesentliche Bindungsdomäne hin verkürzt und durch chemische oder enzymatische Synthese dargestellt werden.

Eine besondere Form von solchermaßen herstellbaren Aptameren sind die sogenannten Spiegelmere, die sich im wesentlichen dadurch auszeichnen, dass sie zumindest teilweise, bevorzugt vollständig aus den nicht-natürlichen L-Nukleotiden aufgebaut sind. Verfahren zur Herstellung derartiger Spiegelmere sind beschrieben in PCT/EP97/04726, deren Offenbarung hiermit durch Bezugnahme aufgenommen wird. Die Besonderheit des darin beschriebenen Verfahrens liegt in der Erzeugung von enantiomeren Nukleinsäuremolekülen, d.h. von L-Nukleinsäuremolekülen, die an ein natives, d.h. in der natürlichen Form oder Konfiguration vorliegendes Target oder eine derartige Targetstruktur binden. Das oben beschriebene in vitro Selektionsverfahren wird dazu eingesetzt, bindende Nukleinsäuren oder Sequenzen zunächst gegen die Enantiomere, d.h. nicht natürlich vorkommenden Struktur eines natürlicherweise vorkommenden Targets zu selektieren, beispielsweise im Falle, dass das Zielmolekül ein Protein ist, gegen ein D-Protein. Die so erhaltenen Bindungsmoleküle (D-DNA, D-RNA bzw. entsprechende D-Derivate) werden in ihrer Sequenz bestimmt und die identische Sequenz wird dann mit spiegelbildlichen Nukleotidbausteinen (L-Nukleotide bzw. L-Nukleotidderivate) synthetisiert. Die so erhaltenen spiegelbildlichen, enantiomeren Nukleinsäuren (L-DNA, L-RNA bzw. entsprechende L-Derivate), sogenannte Spiegelmere, haben aus Symmetriegründen eine spiegelbildliche Tertiärstruktur und somit eine Bindungseigenschaft für das in der natürlichen Form oder Konfiguration vorliegende Target.

Die vorstehend beschriebenen Zielmoleküle, auch als Target bezeichnet, können Moleküle oder Strukturen sein, so z. B. Viren, Viroide, Bakterien, Zelloberflächen, Zellorganellen, Proteine, Peptide, Nukleinsäuren, kleine Moleküle wie Metaboliten des Stoffwechsels, pharmazeutische Wirkstoffe oder deren Metabolite oder andere chemische, biochemische oder biologische Komponenten.

Die Erfindung wird im folgenden anhand der Figuren und Beispiele weiter erläutert, aus denen sich weitere Vorteile, Ausgestaltungsformen und Merkmale der Erfindung ergeben.

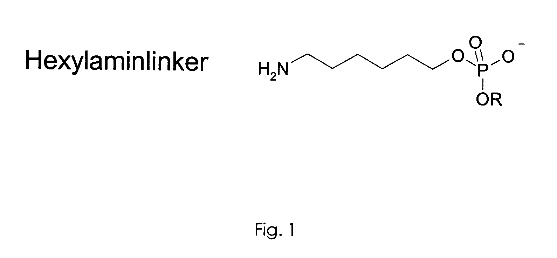

- Fig. 1: zeigt einen Hexylaminlinker, der über einen linearen, aus sechs Kohlenstoffatomen bestehenden Abstandshalter ("spacer") sowie eine terminale Aminogruppe und einen terminalen Phosphatrest verfügt. Die mit R angegebene Substitution kann dabei auch eine Nukleinsäure darstellen bzw. den L-Nukleinsäureteil einer modifizierten L-Nukleinsäure. Über die Amingruppe kann der Nicht-L-Nukleinsäure-Teil an den L-Nukleinsäure-Teil gekoppelt und damit die erfindungsgemäße modifizierte L-Nukleinsäure ausgebildet werden.

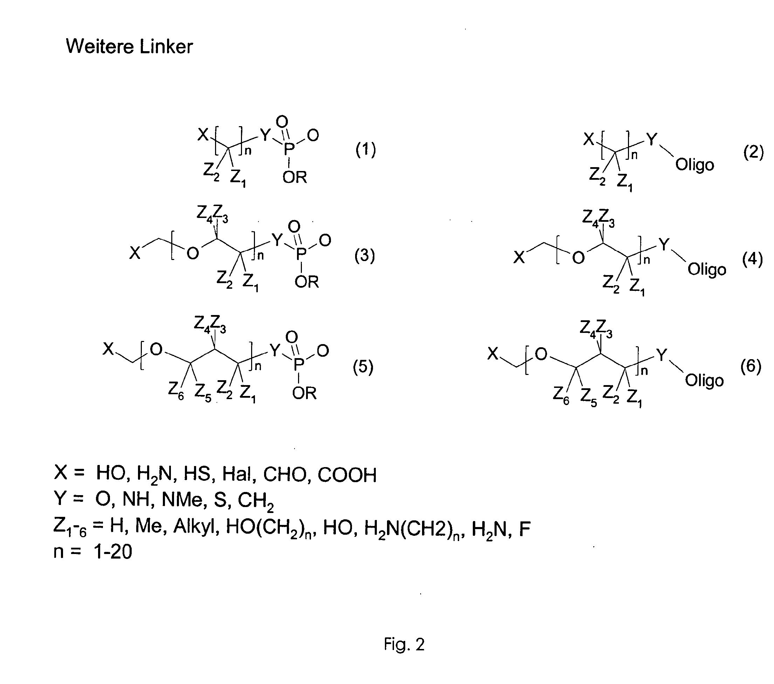

- Fig. 2: zeigt weitere Linker, wobei die mit (2), (4) und (6) bezeichneten Strukturen den Linkern gemäß (1), (3) und (5) entsprechen, wobei bei letzteren jedoch der mit dem Rest R versehene Phosphatteil bevorzugterweise den L-Nukleinsäure-Teil darstellt und über die mit X bezeichnete funktionelle Gruppe der Nicht-L-Nukleinsäure-Teil der modifizierten L-Nukleinsäure an die L-Nukleinsäure gekoppelt wird. Die Bezeichnung "Oligo" steht dabei beispielhaft für ein Oligonukleotid, wobei es auch im Schutzumfang der vorliegenden Erfindung ist, dass es sich dabei bzw. bei den L-Nukleinsäuren oder L-Nukleinsäure-Teilen hierin allgemein um L-Polynukleotide handeln kann. Die verschiedenen Substituenten bezeichnen dabei die folgenden reaktiven Gruppen, die einzeln und jeweils unabhängig voneinander sind:
X = OH, NH₂, HS, Hal, CHO, COOH
Y = O, NH, NMe, S, CH₂
Z₁, Z₂, Z₃, Z₄, Z₅ und Z₆ = H, Me, Alkyl, HO(CH₂)ₙ, HO, H₂N (CH₂)ₙ, H₂N, F,
wobei n eine ganze Zahl zwischen 1 und 20 ist und wobei Alkyl lineare und verzweigte Kohlenwasserstoffketten mit bevorzugterweise 1-20 C-Atomen bevorzugtererweise 1 bis 4 C-Atomen und/oder -(CH₂)ₙH, -CH[(CH₂)ₙH][(CH₂)ₘH], -C[(CH₂)ₙH][(CH₂)ₘH][(CH₂)ₗH], -(CH₂)ₙ(CH)ₘ[(CH₂)ₗH][(CH₂)ₖH], -(CH₂)ₙ(C)ₘ[(CH₂ )ₗH] [(CH₂)ₖH][(CH₂)ⱼH], bezeichnet, wobei n, m, l, k und j unabhängig voneinander ganze Zahlen zwischen 1 und 8, bevorzugterweise 1 bis 4 C-Atome sind.

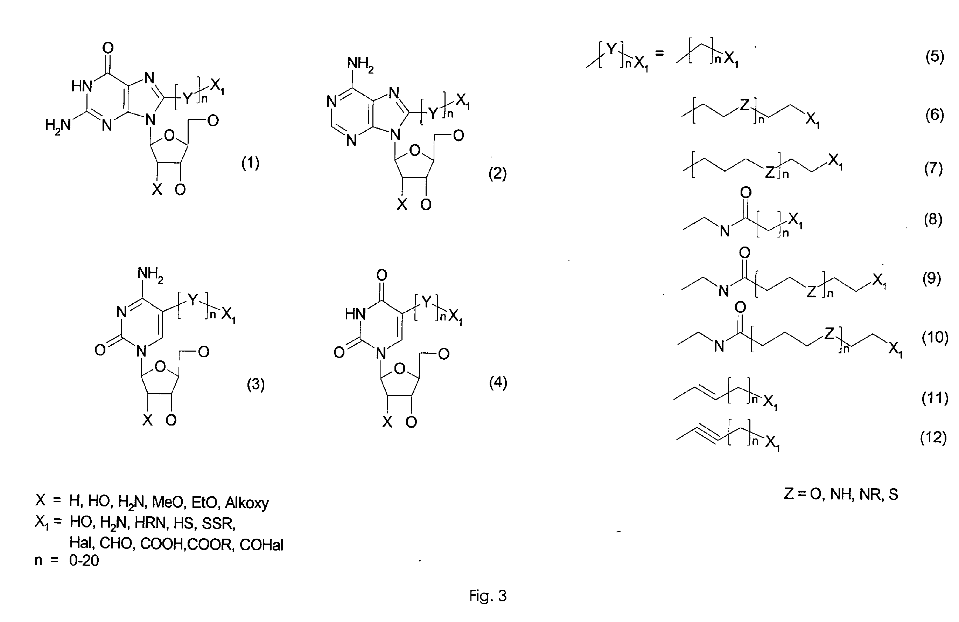

- Fig. 3: zeigt eine Übersicht verschiedener Linker, die an verschiedene Positionen der Nukleobasen gekoppelt sind. Dabei ist beachtlich, dass der Zuckeranteil des jeweils dargestellten Nukleosids eine Ribose, eine Desoxyribose oder eine modifizierte Ribose bzw. modifizierte Desoxyribose sein kann und der Rest X = H, HO, H₂N, MeO, EtOH oder Alkoxy sein kann. Alkoxy bezeichnet dabei insbesondere lineare und verzweigte Oxykohlenwasserstoffketten mit 1-20 C-Atomen, bevorzugterweise 1 bis 4 C-Atomen und/oder -O(CH₂)ₙH, -OCH[(CH₂)ₙH][(CH₂)ₘH], -OC[(CH₂)ₙH][(CH₂)ₘH][(CH₂)ₗH], -O(CH₂)ₙ(CH)ₘ[(CH₂)ₗH][(CH₂)ₖH], -O(CH₂)ₙ(C)ₘ[(CH₂)ₗH] [(CH₂)ₖH][(CH₂)ⱼH], wobei n, m, l, k und j unabhängig voneinander ganze Zahlen zwischen 1 und 20, bevorzugterweise 1 bis 4 C-Atome sind. Die eigentliche Linkerstruktur ist bei allen vier dargestellten Nukleosiden (1), (2), (3) und (4) X₁-[Y]ₙ, wobei n eine ganze Zahl zwischen 0 und 20 ist. X₁ stellt eine funktionelle Gruppe dar, die ausgewählt ist aus der Gruppe, die HO, H₂N, HRN, HS, SSR, Hal, CHO, COOH, COOR und COHal umfasst. Bei den in den Strukturformeln (5-12) angegebenen Linkern ist n ebenfalls eine ganze Zahl zwischen 0 und 20 und Z bedeutet unabhängig von anderen Substituenten, entweder O, NH, NR oder S, wobei R für Alkyl steht, wie hierin definiert.

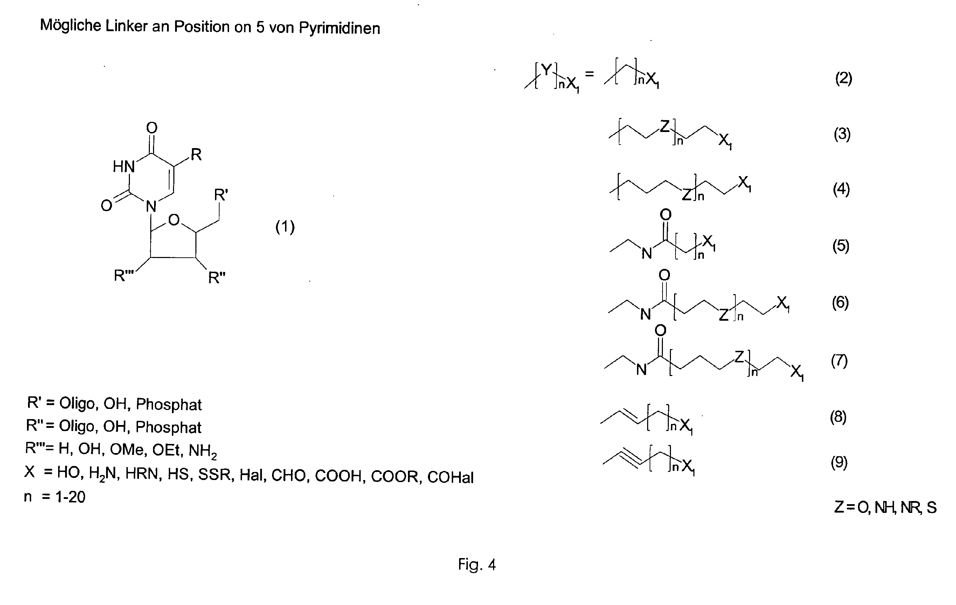

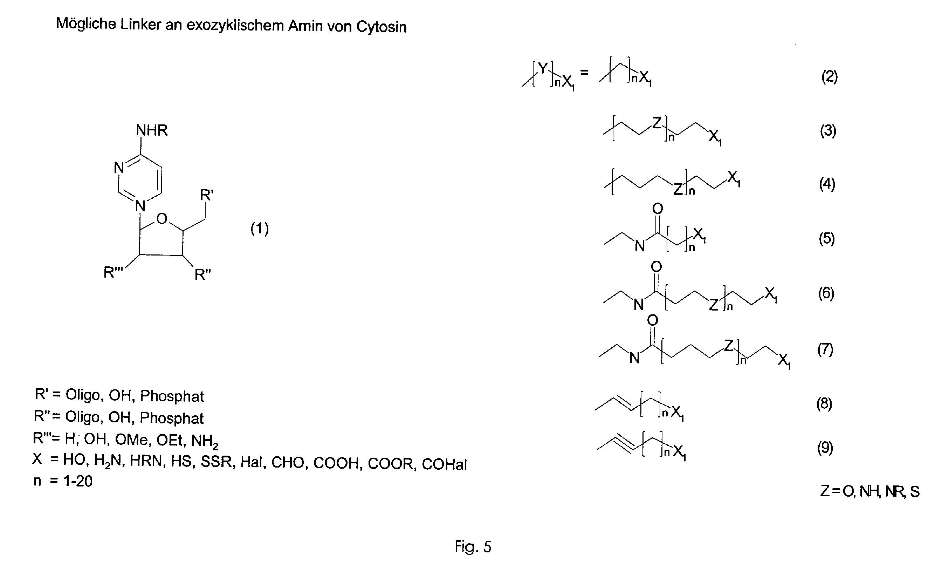

- Fig. 4: zeigt mögliche Linker an Position 5 von Pyrimidin-Nukleosiden bzw. - Nukleotiden. Hinsichtlich der Reste R', R" und R"' gilt das im Zusammenhang mit Fig. 5 Ausgeführte sinngemäß. Der Linker R weist die Struktur -[Y]ₙ-Xₗ auf und kann bevorzugterweise die in den Strukturen (2) bis (9) gezeigten Formen annehmen, wobei auch hier Z unabhängig von der Wahl der anderen Substituenten O, NH, NHR oder S bedeuten kann und n eine ganze Zahl zwischen 1 und 20 sein kann. Die funktionelle Gruppe X₁ ist bevorzugterweise ausgewählt aus der Gruppe, die HO, H₂N, HRN, HS, SSR, Hal, CHO, COOH, COOR, COHal aufweist.
- Fig. 5: zeigt in 1 den grundsätzlichen Aufbau von Cytosin, welches an seinem exocyclischen Amin verschiedene Linkerstrukturen aufweisen kann. Dabei bezeichnet R' eine L-Nukleinsäure oder ein L-Polynukleotid, OH oder Phosphat, R" eine L-Nukleinsäure oder ein L-Polynukleotid, OH oder Phosphat und R''' H, OH, OMe, OEt, NH₂. Der Rest R bezeichnet dabei den Linker, der die Grundstruktur-[Y]ₙ-X₁ aufweist und die in (2) bis (9) gezeigten Strukturformeln aufweisen kann, wobei Z = O, NH, NR, S und n eine ganze Zahl zwischen 1 und 20 sein kann. Die funktionelle Gruppe X₁ ist bevorzugterweise ausgewählt aus der Gruppe, die HO, H₂N, HRN, HS, SSR, Hal, CHO, COOH, COOR, COHal aufweist.

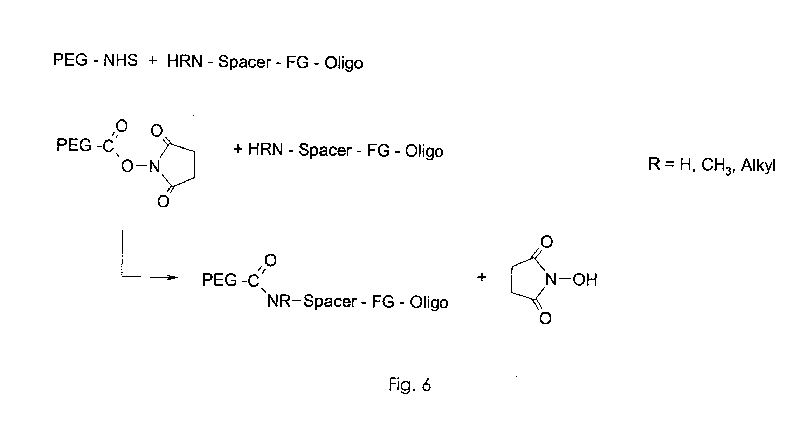

- Fig. 6: zeigt die Ausbildung einer erfindungsgemäß modifizierten L-Nukleinsäure durch Reaktion von PEG-NHS mit einer mit einem Linker versehenen L-Nukleinsäure. Nach erfolgreicher Kopplung liegt die modifizierte L-Nukleinsäure vor, die im vorstehenden Fall als Nicht-L-Nukleinsäure-Teil PEG umfasst und als L-Nukleinsäure in diesem konkreten Falle ein Oligonukleotid, wobei zwischen beiden ein eine Aminogruppe tragender Linker bzw. Spacer zwischengeschaltet ist und es zwischen dem Linker und dem PEG zu einer Säureamidbindung kommt. Neben der modifizierten L-Nukleinsäure wird als weiteres Reaktionsprodukt das von PEG abgespaltene N-Hydroxysuccinimid erhalten. Als mögliche Reste R sind H, CH₃ und allgemein Alkylketten mit einer Länge von 1-20 bevorzugt. Die funktionelle Gruppe kann prinzipiell das Produkt einer jeden der hierin vorstehend ausgeführten Reaktionen sein. Insoweit gelten die dort und in Verbindung mit den weiteren Figuren, insbesondere Fig. 2 und 3, beschriebenen Ausgestaltungen des Linkers auch in diesem Zusammenhang. Gleiches gilt auch für die in der Formel dargestellten Substituenten und Laufvariablen wie n.

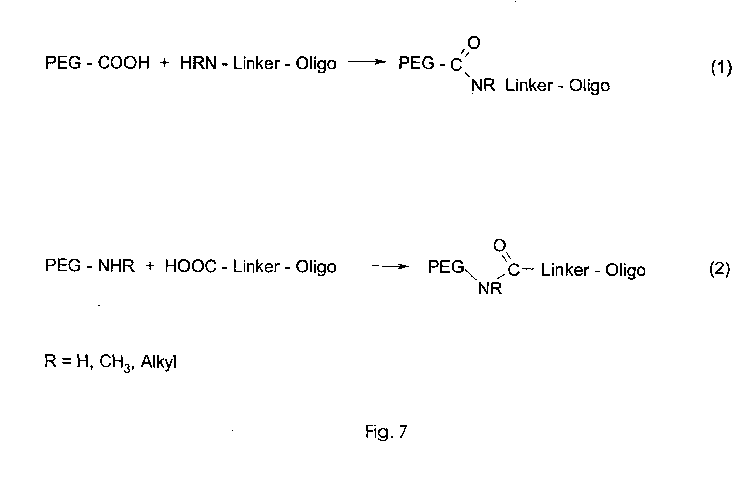

- Fig. 7: zeigt die Umsetzung verschiedener PEG-Derivate mit verschiedenen Linkern. Dabei unterscheiden sich die beiden Reaktionen (1) und (2) lediglich darin, dass bei Reaktion (1) die Carboxylgruppe am PEG und in Reaktion (2) die Carboxylgruppe an einem mit einem Linker versehenen L-Oligonukleotid vorhanden ist. Bei der funktionellen Gruppe des jeweils korrespondierenden Reaktionspartners, d. h. im Falle der Reaktion (1) der mit einem Linker versehenen L-Nukleinsäure, im Falle der Reaktion (2) der mit einer Amingruppe versehenen PEG. Damit wird die im Zusammenhang mit den verschiedenen oben angeführten Reaktionen, die zwischen L-Nukleinsäure-Teil und Nicht-L-Nukleinsäure-Teil, ggf. unter Beteiligung eines oder mehrerer zwischengeschalteter Linker, möglich sind, gemachte Aussage bestätigt, dass grundsätzlich die genannten reaktiven Gruppen bei allen beteiligten Reaktionspartnern vorhanden sein können. Die letzten Endes erhaltenen Strukturen werden sich entsprechend unterscheiden, so im Falle der Reaktion (1), wo eine Säureamidgruppe am PEG vorhanden ist, und im Falle der Reaktion (2), wo die Säureamidbindung am Konstrukt aus Linker und Oligonukleotid, d. h. L-Nukleinsäure, vorhanden ist. Hinsichtlich der Substituenten R gilt das im Zusammenhang mit Fig. 6 Gesagte entsprechend.

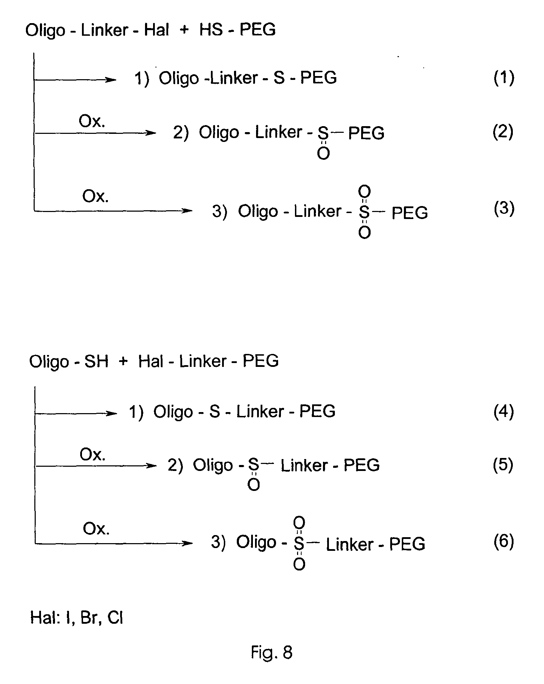

- Fig. 8: zeigt die Reaktion eines Halogenids mit einem Thioester, die entweder am Nicht-L-Nukleinsäure-Teil bzw. am L-Nukleinsäure-Teil angebracht sind. Bei den Reaktionen (1) bis (3) ist dabei vorgesehen, dass die L-Nukleinsäure, hier wie bei allen Figuren verkürzend als oligo bezeichnet, mit einem Linker versehen ist und der Linker ein Halogenid, wie beispielsweise I, Br, Cl trägt. Diese derivatisierte L-Nukleinsäure wird sodann mit einem mit einer Thiolgruppe, bevorzugt einer terminalen Thiolgruppe, versehenen PEG umgesetzt. Im Falle der Reaktion (1) kommt es dabei zu einer Thioetherbindung zwischen Linker und PEG. Durch Oxidation kann es, wie in Reaktion (2) dargestellt, zur Ausbildung eines Sulfoxids bzw. eines Sulfons kommen. Bei den Reaktionen gemäß (4) bis (6) erfolgt ebenfalls eine Umsetzung zwischen einem Thiol und einem Halogenid, wobei in diesen Fällen die L-Nukleinsäure mit der Thiolgruppe versehen ist und der Linker das Halogenid trägt. Entsprechend kommt es zur Ausbildung von Verbindungen, bei denen der Schwefel zwischen der L-Nukleinsäure und dem Linker angeordnet ist und dieser, wie in den Reaktionen (5) und (6) dargestellt, wiederum zu den entsprechenden Derivaten oxidiert werden kann.

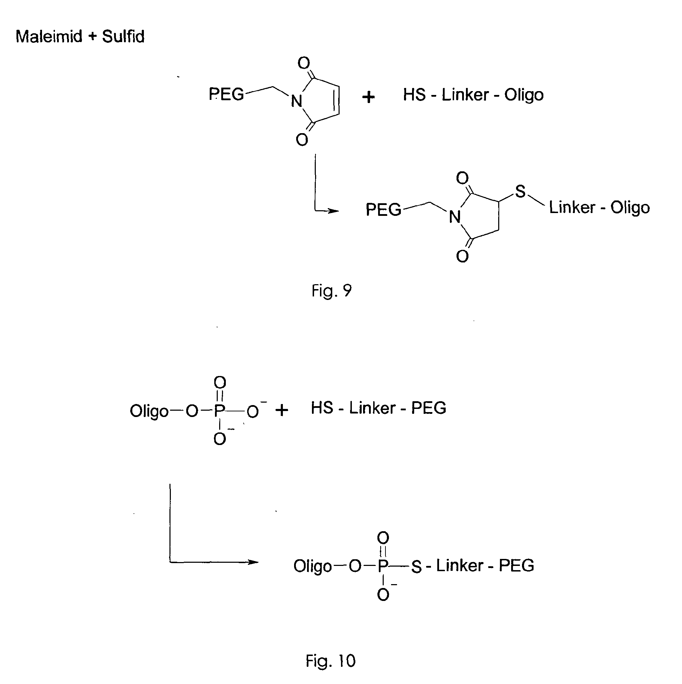

- Fig. 9: zeigt die Reaktion des mit einer Maleimid-Gruppe versehenen PEG mit einer L-Nukleinsäure, dort als oligo bezeichnet, die einen eine Thiol-Gruppe tragenden Linker aufweist. Bei dem Reaktionsprodukt handelt es sich um einen Thioether.
- Fig. 10: zeigt die Reaktion einer Phosphat-tragenden L-Nukleinsäure mit einem PEG, das mit einem eine Thiol-Gruppe tragenden Linker versehenen ist. Das Reaktionsprodukt ist ein Phosphothioat.

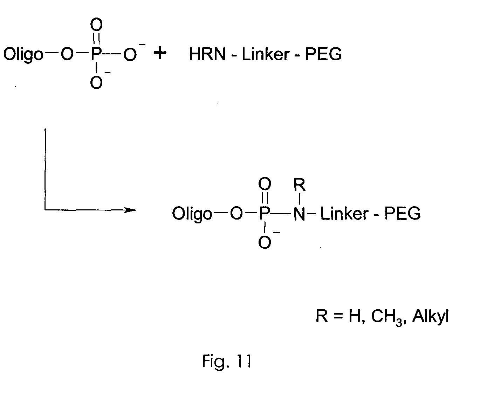

- Fig. 11: zeigt die Reaktion einer mit einem Phosphatrest - ggf. terminal - versehenen L-Nukleinsäure mit einem PEG, das mit einem ein Amin aufweisenden Linker versehenen ist. Bei dem Reaktionsprodukt handelt es sich um ein Phosphoramidat. Hinsichtlich des Restes R gilt das im Zusammenhang mit Fig. 6 Ausgeführte.

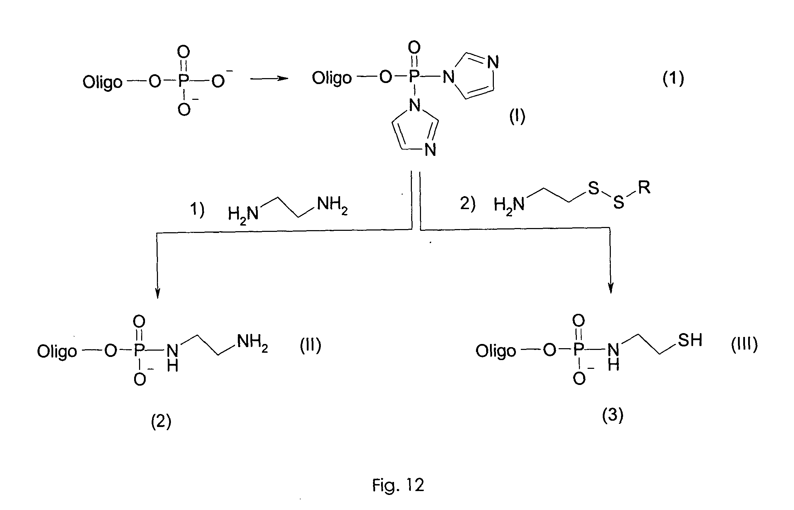

- Fig. 12: zeigt den Einbau einer reaktiven Amino- bzw. Thiolgruppe in eine L-Nukleinsäure unter Verwendung einer aktivierten Phosphatgruppe, bevorzugterweise einer terminalen Phosphatgruppe der L-Nukleinsäure. Hierbei wird in einem ersten Schritt ein Phosphorimidazolid (I) hergestellt, welches unter Verwendung eines Ethylendiamins im Falle der Reaktion (2) zur Ausbildung eines 2-Aminoethylen-1-phophoramidat (II) bzw. im Falle der Reaktion (3) unter Verwendung von Cysteamin zu 2-Thioethylen-1-phophoramidat (III) führt. Die Verbindungen gemäß (II) und (III) können sodann mit Nicht-L-Nukleinsäuren, insbesondere den hierin offenbarten Verbindungen, umgesetzt werden.

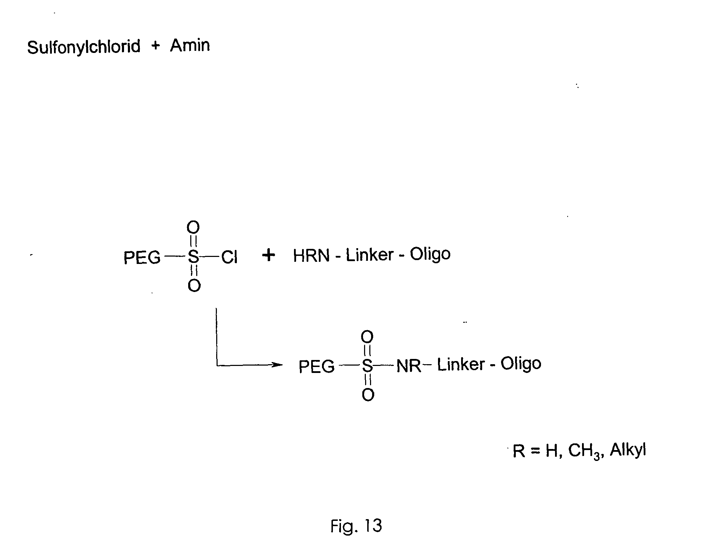

- Fig. 13: zeigt die Reaktion eines mit einer Sulfonylchlorid-Gruppe versehenen PEGs mit einer L-Nukleinsäure, die einen Linker aufweist, der eine Amingruppe trägt. Das Reaktionsprodukt ist ein Sulfonamid. Hinsichtlich des Restes R gilt das im Zusammenhang mit Fig. 6 Ausgeführte.

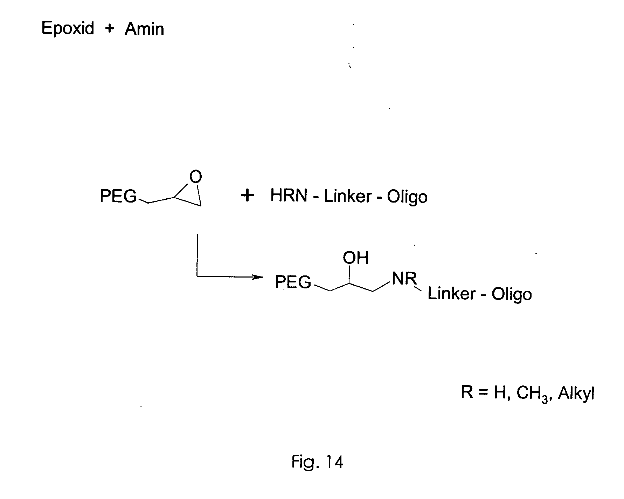

- Fig. 14: zeigt die Reaktion eines mit einer Epoxid-Gruppe versehenen PEG mit einer L-Nukleinsäure, welche einen mit einer Amin-Gruppe versehenen Linker aufweist unter Ausbildung eines Amins. Hinsichtlich des Restes R gilt das zu Fig. 6 Ausgeführte.

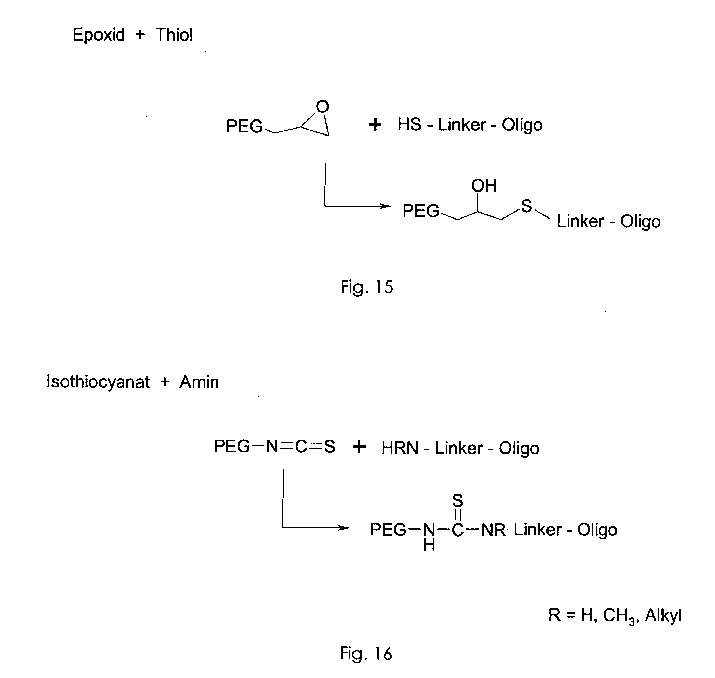

- Fig. 15: zeigt die Reaktion eines mit einer Epoxid-Gruppe versehenen PEG mit einer L-Nukleinsäure, die einen eine Thiol-Gruppe aufweisenden Linker trägt. Bei dem Reaktionsprodukt handelt es sich um einen Thioether.
- Fig. 16: zeigt die Reaktion eines eine Isothiocyanat-Gruppe aufweisenden PEG mit einer L-Nukleinsäure, die einen eine Amin-Gruppe aufweisenden Linker trägt. Das Reaktionsprodukt ist ein Isothioharnstoff. Hinsichtlich des Restes R gilt das im Zusammenhang mit Fig. 6 Ausgeführte.

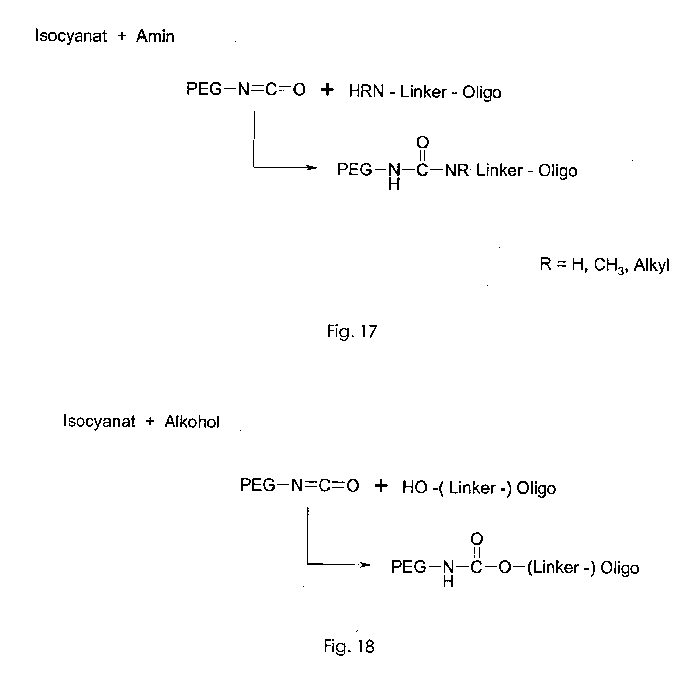

- Fig. 17: zeigt die Reaktion eines mit einer Isocyanat-Gruppe versehenen PEG mit einer L-Nukleinsäure, die einen eine Amin-Gruppe tragenden Linker aufweist, unter Ausbildung eines Isoharnstoffes. Hinsichtlich des Restes R gilt das im Zusammenhang mit Fig. 6 Ausgeführte.
- Fig. 18: zeigt die Reaktion eines mit einer Isocyanat-Gruppe versehenen PEG mit einer L-Nukleinsäure, die eine freie OH-Gruppe trägt, die direkt von der L-Nukleinsäure, wie beispielsweise einer Phosphatgruppe oder dem Zuckerteil des Nukleosids, d. h. den Positionen 2'-OH, 3'-OH oder 5'-OH stammen kann. Alternativ kann die OH-Gruppe an die L-Nukleinsäure über einen geeigneten Linker verknüpft sein. Bei dem Reaktionsprodukt handelt es sich um ein Carbamat.

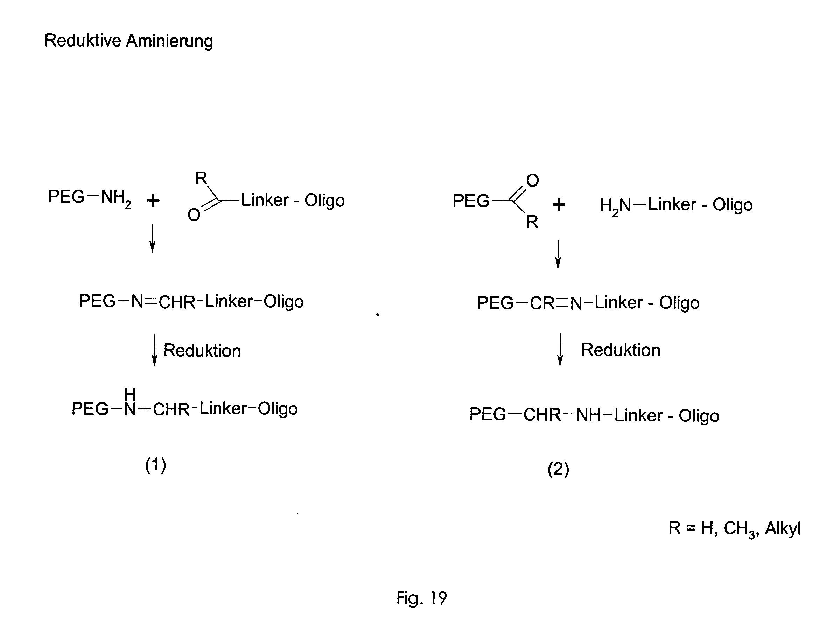

- Fig. 19: zeigt die Reaktion einer Aldehyd- oder Keto-Gruppe mit einer Amino-Gruppe, die jeweils entweder an dem Nicht-L-Nukleinsäure-Teil (Reaktion (1)), im dargestellten Falle an PEG, oder an dem L-Nukleinsäure-Teil (Reaktion (2) vorhanden ist. Der L-Nukleinsäure-Teil weist dabei bevorzugterweise einen Linker auf, der die jeweilige reaktive Gruppe, d. h. die Amino-Gruppe oder die Carbonyl-Gruppe, trägt. Im Falle der Reaktion (1) trägt das PEG die Amino-Gruppe, wohingegen die L-Nukleinsäure einen Linker aufweist, der eine Carbonyl-Gruppe trägt. Das unmittelbar erhaltene Reaktionsprodukt Imin wird sodann durch Reduktion in ein Amin überführt. Im Falle der Reaktion (2) wird das eine Carbonyl-Gruppe tragende PEG mit einer L-Nukleinsäure umgesetzt, die einen Linker trägt, der eine Amino-Gruppe aufweist. Das Reaktionsprodukt Imin wird reduziert und führt zu einem Amin. Hinsichtlich des Restes R gilt das zu Fig. 6 Ausgeführte.

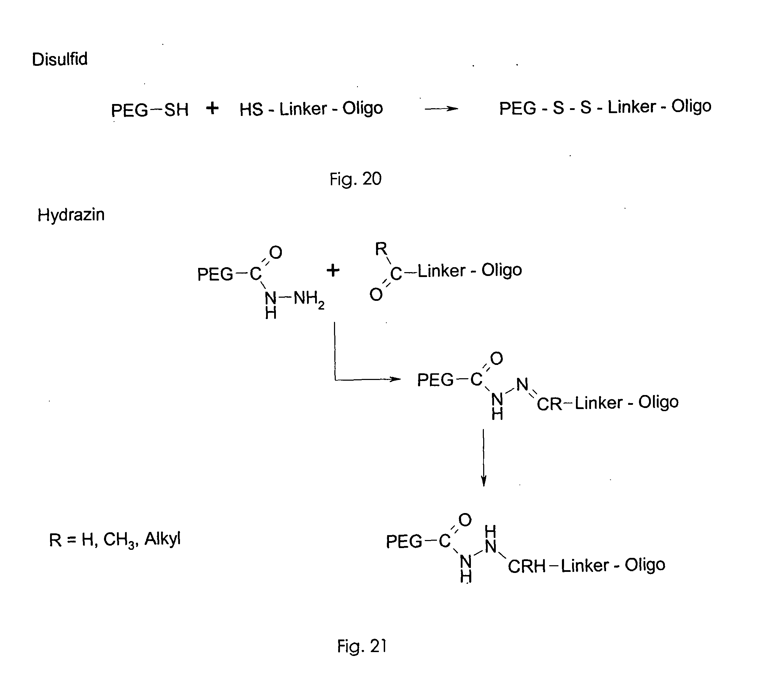

- Fig. 20: zeigt die Reaktion eines mit einer Thiol-Gruppe versehenen PEG mit einer L-Nukleinsäure, die einen ebenfalls mit einer Thiol-Gruppe versehenen Linker trägt. Bei dem Reaktionsprodukt handelt es sich um eine modifizierte L-Nukleinsäure, die über eine Disulfid-Gruppe zwischen dem PEG und der L-Nukleinsäure, genauer gesagt dem daran gebundenen Linker, verfügt.
- Fig. 21: zeigt die Reaktion eines mit einer Hydrazin-Gruppe versehenen PEG mit einer L-Nukleinsäure, die einen eine Carbonyl-Gruppe umfassenden Linker trägt. In einem ersten Reaktionsschritt wird ein Hydrazon erhalten, welches sodann reduktiv in ein substituiertes Hydrazin überführt wird. Hinsichtlich des Restes R gilt das im Zusammenhang mit Fig. 6 Ausgeführte.

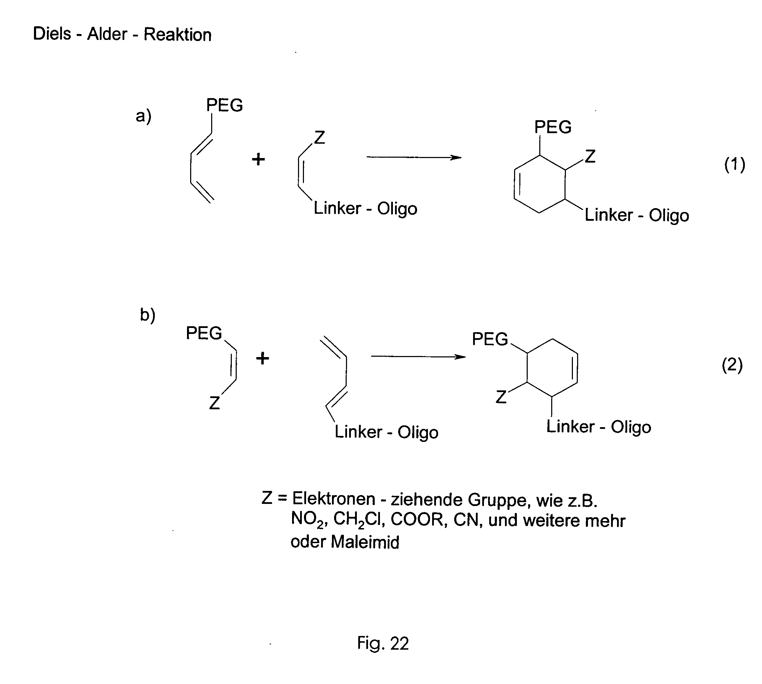

- Fig. 22: zeigt in Reaktion (1) die Umsetzung eines mit einem konjugierten Dien versehenen PEG mit einer L-Nukleinsäure, die einen Linker mit einer sogenannte dienophile Gruppe trägt. Das Dienophil besteht aus einer C-C-Doppelbindung, die wiederum einen Substituenten Z aufweist, der eine elektronenziehende Gruppe umfasst. Dabei kann es sich bevorzugterweise um NO₂, CH₂Cl, COOR, CN oder Maleimid handeln. Hinsichtlich des Restes R gilt das zu Fig. 6 Ausgeführte. Infolge dieser Reaktionen kommt es zur Ausbildung einer modifizierten L-Nukleinsäure, die zwischen dem PEG und der mit einem Linker versehenen L-Nukleinsäure eine Hexenyl-Gruppe. Die in Reaktion (2) gezeigte Diels-Alder-Reaktion geht von einem PEG aus, welches ein Dienophil mit dem Substituenten Z aufweist, das mit einer L-Nukleinsäure, die einen Linker umfasst, der ein konjugiertes Dien trägt. Hinsichtlich des Substituenten Z gilt das im Zusammenhang mit Reaktion (1) Gesagte. Das Reaktionsprodukt ist bei dieser Reaktion (2) ebenfalls ein über eine Hexenyl-Gruppe verbundenes L-Nukleinsäure-Konjugat.

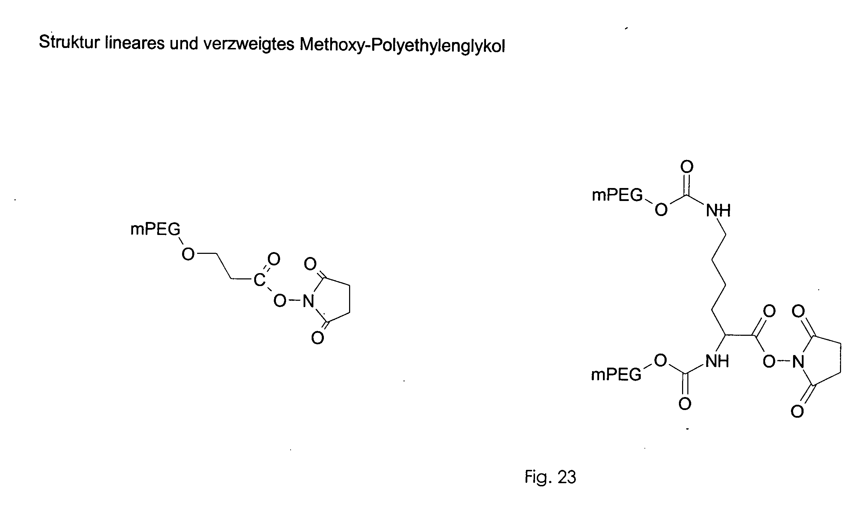

- Fig. 23: zeigt die Struktur der verwendeten verzweigten und linearen mPEG-NHS-Ester.
- Fig. 24: zeigt in (1) den grundsätzlichen Aufbau eines abasischen L-Nukleosides, welches anstelle der Nukleobase entweder ein Wassertstoffatom oder eine oder mehrere, optional verschiedene, Linkerstrukturen aufweisen kann. Dabei bezeichnet R' eine L-Nukleinsäure oder ein L-Polynukleotid, OH oder Phosphat, R" eine L-Nukleinsäure oder ein L-Polynukleotid, OH oder Phosphat und X = H, OH, OMe, OEt, NH₂. Der Rest R bezeichnet entweder das Wasserstoffatom anstelle der Nukleobase oder den Linker, der die in (2) bis (8) gezeigten Strukturformeln aufweisen kann, wobei Z = CH₂, O, NH, NR, S und n eine ganze Zahl zwischen 1 und 20 sein kann. Die funktionelle Gruppe X₁ ist bevorzugterweise ausgewählt aus der Gruppe, die HO, H₂N, HRN, HS, SSR, Hal, CHO, COOH, COOR, COHal aufweist.

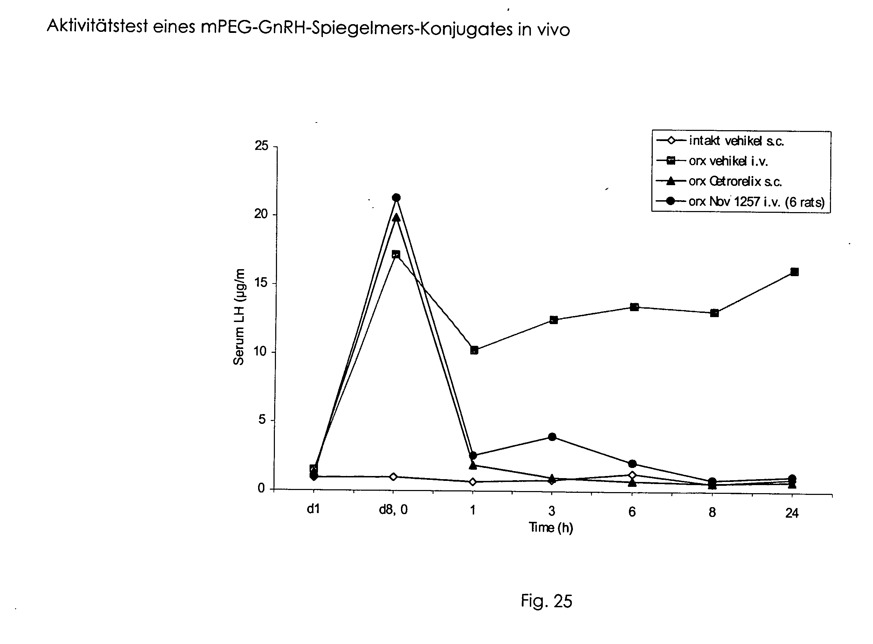

- Fig. 25: zeigt einen Aktivitätstest eines PEG-GnRH-Spiegelmers-Konjugates in männlichen, orchidektomierten Ratten.

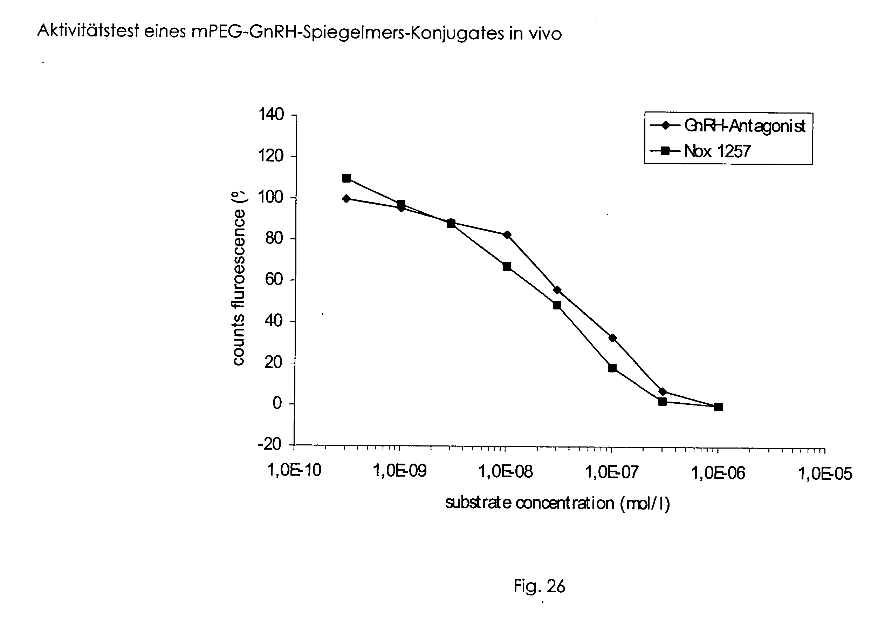

- Fig. 26: zeigt einen Aktivitätstest eines PEG-GnRH-Spiegelmer-Konjugates in vitro in CHO-Zellen.

### Beispiel 1: Synthese von L-Nukleinsäure-PEG-Konjugaten

Die Bedingungen für die Synthese von L-Nukleinsäure-PEG-Konjugaten ausgehend von der in SEQ ID NO:2 dargestellten L-Nukleinsäure und PEG, wobei das PEG dergestalt modifiziert war, dass es entweder als NHS-Ester oder als primäres Amin zur Kopplung an ein Amin bzw. ein Phosphat vorlag, wurden untersucht. Dabei wurde so vorgegangen, dass die Nukleinsäure in einem wässrigen System gelöst wurde. Der pH wurde durch verschiedene Puffer oder Basen wie zum Beispiel NaHCO₃, NaH₂PO₄/Na₂HPO₄, HEPES, MOPS, NH₄OAc, Triethylamin auf pH 6.5 - 9.0 eingestellt. Der Einfluss eines Zusatzes von verschiedenen organischen Lösungsmittel wie zum Beispiel DMF, DMSO, Acetonitril und anderen wurde ausgetestet, wobei der Anteil des organischen Lösungsmittels zwischen 0-100% variiert wurde. Anschliessend erfolgte die Zugabe von verschiedenen PEG-Derivaten wie zum Beispiel verzweigtem mPEG₂-NHS-Ester, linearem mPEG-NHS-Ester oder mPEG-NH₂ (Shearwater Corporations) verschiedener Molekulargewichte zwischen 10.000 Da und 40.000 Da. Die Zugabe von PEG-NHS-Ester kann auf unterschiedliche Weisen erfolgen. So kann beispielsweise PEG-NHS-Ester in einer niedrig konzentrierten Säure wie zum Beispiel in 0,01 N HCl gelöst oder in einem organischen Lösungsmittel wie zum Beispiel DMF gelöst langsam zugetropft oder als Feststoff zugegeben werden. Die bevorzugte Form der Zugabe von PEG-NHS ist portionsweise als Feststoff. Weiter wurde der Einfluss der Reaktionstemperatur zwischen 4°C-65°C getestet. Als Nukleinsäuren wurden Nukleinsäuren mit den folgenden Sequenzen verwendet. 5'-NH₂-TAT TAG AGA C -3' (SEQ ID NO:2), und 5'-PO₄-TAT TAG AGA C -3' (SEQ. ID. No 3) sowie die Nukleinsäure gemäß SEQ. ID. No. 1. Die Ausbeuten der oben zusammengefassten Reaktionen lagen zwischen 5 - 78%.

Die bevorzugte Reaktionsvariante war die insgesamt sechsmalige Zugabe von je zwei Äquivalenten festem PEG-NHS-Ester im Abstand von etwa 30 Minuten zu einer Nukleinsäure gelöst in einem Lösungsmittel bestehend aus 60 Teilen H₂O und 40 Teilen DMF unter Zusatz von NaHCO₃ (0,2 M), einem pH von 8,0 und 37°. Diese Reaktionsbedingungen führten zu einer Ausbeute von 78 %.

### Beispiel 2: Synthese eines L-Nukleinsäure-Phosphoramidat-PEG-Konjugates

Ausgehend von einer L-Nukleinsäure mit der Sequenz 5'-PO₄-TAT TAG AGA C-3' (SEQ ID NO:3) wurde ein entsprechendes Phosphoramidat-PEG-Konjugat hergestellt. Die L-Nukleinsäure (10 OD) wurde mit PEG-NH₂ (20.000 Da, linear, 1 - 10 Äquivalente) in wässriger Lösung mit EDCI bei 50°C zu einem L-Nukleinsäure-Phosphoramidat-PEG-Konjugat umgesetzt. Die Analyse und Aufreinigung verlief analog zu der PEGylierung von L-Nukleinsäuren mit PEG-NHS, wie in Beispiel 1 beschrieben. Die Reaktionsbedingungen wurden nicht optimiert und führten zu einer Ausbeute von < 8 %.

### Beispiel 3: PEGylierung eines GnRH-Spiegelmer-Liganden

Das Peptidhormon GnRH I (Gonadotropin Releasing Hormon, Gonadoliberin), welches allgemein auch als GnRH bezeichnet wird, ist ein Deka-Peptid, welches im Hypothalamus gebildet wird und die Sekretion der Gonadotropin-Hormone luteinisierendes Hormon (LH) und Follikel-stimulierendes Hormon (FSH) durch die Hypophyse stimuliert. GnRH wird aus den Neuronen des Hypothalamus in pulsierender Form sezerniert und bindet dann an seinen Rezeptor auf der Zelloberfläche der Hypophyse. Der Ligand-Rezeptor-Komplex wird internalisiert, wodurch es zu einer Ausschüttung von FSH und LH kommt, welche wiederum die Bildung von Sexualhormonen wie Östrogen, Progesteron oder Testosteron anregen. Es konnte ein Spiegelmer, d. h. eine L-Nukleinsäure, erzeugt werden, die spezifisch an GnRH bindet und die folgende Sequenz aufweist:

5' - CCA AGC TTG CGT AAG CAG TCT CCT CTC AGG GGA GGT TGG GCG GTG
CGT AAG CAC CGG TTT GCA GGG G - 3' (SEQ ID NO:1)

Die Synthese des Spiegelmers mit der vorstehend aufgezeigten Sequenz wurde auf einem Amersham Pharmacia Biotech Oligopilot II DNA-Synthesiser im 780-µMol Maßstab an einer 1000Å CPG-Festphase (Controlled Pored Glass) nach der 2-Cyanoethyl-Phosphoramidit-Chemie (Sinha et al. NAR, 12, 1984, p. 4539ff) durchgeführt. Anschließend wurde 6-(Monomethoxytritylamino)-hexyl-(2-cyanoethyl)-(N,N-diiospropyl)-phosphoramidit an das 5'-Ende des Spiegelmeres (5'-MMT-Aminohexyl-Spiegelmer) gekoppelt, um die postsynthetische Konjugation mit PEG zu ermöglichen.

Nach Beendigung der Synthese wurde das 5'-MMT-Aminohexyl-Spiegelmer durch 8-stündige Inkubation in 33%iger Ammoniaklösung bei 65°C von der Festphase abgespalten und komplett entschützt, danach zur Trockene eingeengt, in 10mM NaOH aufgenommen und mittels RP-HPLC gereinigt. Die Abspaltung der Monomethoxytrityl-Schutzgruppe erfolgte mit 0.4% Trifluoressigsäure (TFA) in 30 min bei RT. TFA wurde durch zweimalige Koevaporation mit Ethanol entfernt und das 5'-Aminohexyl-Spiegelmer gemäß SEQ. ID. No.1 wurde durch Fällung in Ethanol gereinigt (Ausbeute: 5,000 OD, 7.5 µmol). Der Produktpeak wurde aufgesammelt und mittels Größenausschlußchromatographie über eine Sephadex G10 Säule oder durch Ultrafiltration (Labscale TFF System, Millipore) entsalzt.

Das solchermaßen 5'-Amino-modifizierte GnRH-Spiegelmer (5,000 OD, 7.5 µmol) wurde in 0.2 M NaHCO₃, pH 8.5 / DMF 60:40 (v/v) (125 mL) vorgelegt, auf 37°C erwärmt und portionsweise mit pulverförmigem N-Hydroxysuccinimidyl (NHS) - aktiviertem Ester von verzweigtem 40,000 Da Poly(ethylen)glykol versetzt (2 eq (Äquivalente) alle 30 min zugeben, insgesamt 12 eq, 6 x 600 mg, 180 µmol). Der Reaktionsverlauf wurde durch analytische Gelektrophorese (8% Polyacrylamid, 8,3 M Harnstoff) verfolgt. Das Rohprodukt wurde zunächst durch Ionenaustausch-HPLC von überschüssigem PEG gereinigt (Source Q 30; Laufmittel A: H₂O, Laufmittel B: 2 M NaCl; Flussrate 20 mL/min; Beladen der Säule und Eluieren von freiem PEG mit 10% B; Elution des PEG-GnRH-Spiegelmer-Konjugates mit 50% B), anschließend wurde durch RP-HPLC PEGyliertes GnRH-Spiegelmer von nicht-PEGyliertem GnRH-Spiegelmer getrennt (Source RPC 15; Laufmittel A: 100 mM Triethylammonium acetat (TEAA), Laufmittel B: 100 mM TEAA in H₂O/Acetonitril 5:95; Flussrate 40 mL/min; Beladen der Säule mit 10% B; Gradient von 10% auf 70% B in 10 Säulenvolumen, Elution von PEG-GnRH-Spiegelmer bei 45-50% B), umgesalzt (Source Q 30; Laufmittel A: H₂O, Laufmittel B: 2 M NaCl; Flussrate 20 mL/min; Beladen der Säule und Eluieren von freiem PEG mit 10% B; Elution von PEG-GnRH-Spiegelmer mit 50% B) und anschließend durch Gelfiltration (Sephadex G10; Laufmittel H₂O; Flussrate 5 mL/min) oder Ultrafiltration (Labscale TFF System, Millipore) entsalzt. Durch Lyophilisation erhielt man das gewünschte Produkt als weißes Pulver (3,900 OD, 375 mg, 78%).

In analoger Weise wurden weitere L-Nukleinsäuren, einschließlich der Sequenz gemäß SEQ.ID.No. 1 mit verschiedenen PEG (linear 10.000 Dalton, linear 20.000 Dalton, verzweigt 20.000 Dalton, linear 35.000 Dalton), verknüpft und aufgereinigt.

### Beispiel 4: Synthese von L-Nukleinsäure-FITC-Konjugaten: Kopplung von Fluorescein-Isothiocyanat an GnRH-Spiegelmer mit einem 5'-NH₂-C₆-Linker

Das gemäß Beispiel 3 hergestellte 5'-Amino-modifizierte GnRH-Spiegelmer wurde in 0.5 M NaHCO₃ pH 8.5 vorgelegt, auf 65°C erwärmt und ein Überschuss Fluorescein-Isothiocyanat (FITC, 10 eq) wurde zum Reaktionsgemisch hinzugegeben. Die Reaktion wurde mittels analytischer RP-HPLC verfolgt. Es wurde für 48 h bei 65°C geschüttelt, überschüssiges FITC durch Centri-Spin10 (Princeton Separations) abgetrennt und mit Fluorescein markierte L-Nukleinsäure mit RP-HPLC aufgereinigt. Lyophilisation lieferte das gewünschte Produkt als gelbliches Pulver in quantitativer Ausbeute.

### Beispiel 5: Aktivitätstest des PEG-GnRH-Spiegelmers in vivo in männlichen, orchidektomierten Ratten

Männliche Ratten wurden orchidektomiert, wodurch der LH-Pegel der Ratten infolge des fehlenden Testosteron-Feedback-Signals über die folgenden acht Tage kontinuierlich anstieg. Am Tag 8 wurde das PEG-GnRH-Spiegelmer, d. h. das Konjugat aus PEG und GnRH-Spiegelmer, sieben Ratten (150 mg/kg) intravenös verabreicht. Blutproben wurden am Tag 0 (vor der Orchidektomie), am Tag 8 (0 Stunden vor i. v. Applikation des PEG-GnRH-Spiegelmers), sowohl 0,5 Std., 1,5 Std., 3 Std., 6 Std. als auch 24 Std. nach der i. v. Applikation entnommen und der jeweilige LH-Spiegel mittels Radioimmunoassay (RIA) bestimmt. Parallel hierzu wurde sieben männlichen, orchidektomierten Ratten nur das Vehikel (PBS-Puffer, pH 7,4) i. v. als Negativ-Kontrolle und sieben männlichen orchidektomierten Ratten der Standard-Antagonist Cetrorelix (100 µg/kg) als Positiv-Kontrolle s. c. verabreicht. Das Ergebnis ist in Fig. 25 dargestellt.

Mit Ausnahme der Negativ-Kontrolle (in Fig. 25 mit Dreiecken bezeichnet) kommt es unter der Einwirkung des PEG-GnRH-Spiegelmers auch nach 24 Stunden noch zu einem LH-Titer, der vergleichbar ist demjenigen von nicht-orchidektomierten Ratten bzw. solchen Ratten, denen man den Standard-Antagonisten Cetrorelix verabreicht hatte. Dies belegt die Eignung des PEG-GnRH-Spiegelmers, die Wirkung des GnRH über einen langen Zeitraum nachhaltig zu beeinflussen. Dass die vorstehend beschriebene Wirkung des PEG-GnRH-Spiegelmers auf die PEGylierung des GnRH-Spiegelmers zurückgeht, ergibt sich aus der Tatsache, dass bei Applikation des GnRH-Spiegelmers ohne entsprechende Modifizierung bei subcutaner Applikation von 100 mg/kg bereits nach wenigen Stunden eine Abnahme der Aktivität des GnRH-Spiegelmers beobachtet werden konnte.

### Beispiel 6: Aktivitätstest von PEG-GnRH-Spiegelmer-Konjugat in vitro in CHO-Zellen

Die hierin beschriebene Zellkulturstudie wurde an Chinese Hamster Ovary (CHO)-Zellen durchgeführt, welche den Humanrezeptor für GnRH exprimieren. Dabei wurde die intrazelluläre Freisetzung von Ca²⁺-Ionen gemessen, da diese für die Signaltransduktion wichtige Freisetzung nach Bildung des Agonist-Rezeptor-Komplexes erfolgt. Der Ca²⁺-Spiegel wurde dann durch einen Ca²⁺-sensitiven Fluoreszenzfarbstoff bestimmt. Das PEG-GnRH-Spiegelmer bzw. das GnRH-Spiegelmer sollte den Agonisten GnRH abfangen und dadurch dessen Bindung an den Rezeptor auf die Zellmembran inhibieren. Dabei wurde experimentell so vorgegangen, dass der Agonist GnRH (2 nM) für 20 min mit dem GnRH-Spiegelmer bzw. mit dem PEG-GnRH-Spiegelmer in einem Konzentrationsbereich von 100 pM bis 1 µM präinkubiert wurde. Diese Lösung wurde sodann jeweils zu den mit Fluoreszenzfarbstoff beladenen CHO-Zellen gegeben und die jeweilige Ca²⁺-Konzentration an einem Fluroescence Imaging Plate Reader (FLIPR) bestimmt. Das Ergebnis ist in Fig. 26 dargestellt.

Ein Standardantagonist wurde als Positiv-Kontrolle verwendet (ausgefüllte Kreise). Die konzentrationsabhängige Bestimmung ergab eine sigmoidale Aktivitätskurve, die darauf hinweist, dass sowohl das native, d. h. nicht modifizierte GnRH-Spiegelmer, wie auch das mit PEG modifizierte GnRH-Spiegelmer die Bildung des GnRH-Rezeptor-Komplexes zu 100 % inhibieren konnte mit einem IC₅₀ von 20 nM für das GnRH-Spiegelmer und 30 nM für das PEG-GnRH-Spiegelmer.

Die im folgenden angegebene Literaturstellen entsprechen den hierin angegebenen, mit hochgestellten Nummern versehenen Literaturhinweisen.

### Literatur

1. Bragg, P. D. & Hou, C. Subunit composition, function, and spatial arrangement in the Ca2+-and Mg2+-activated adenosine triphosphatases of Escherichia coli and Salmonella typhimurium. *Arch Biochem Biophys* **167**, 311 (1975).
2. Lomant, A. J. & Fairbanks, G. Chemical probes of extended biological structures: synthesis and properties of the cleavable protein cross-linking reagent [35S]dithiobis(succinimidyl propionate). *J Mol Biol* **104**, 243-261 (1976).
3. Buter, J. & Kellogg, R. M. Synthesis of macrocyclic and medium ring dithia compounds using cesium thiolates. *J*. *Chem. Soc. Chem. Commun.,* 466-468 (1980).
4. Buter, J. & Kellogg, R. M. Synthesis of sulfur-containing macrocycles using cesium thiolates. *J Org. Chem.* **46**, 4481-4485 (1981).
5. Kennedy, R. J. & Stock, A. M. The oxidation of organic substances by potassium peroxymonosulfates. *J*. *Org. Chem.* **25**, 1901-1906 (1960).
6. Huang, Z., Schneider, K. C. & Benner, S. A. Oligonucleotide analogs with dimethylenesulfide, -sulfoxide, and -sulfone groups replacing phosphodiester linkages. *Methods Mol Biol* **20**, 315-353 (1993).
7. Connolly, B. A. & Rider, P. Chemical synthesis of oligonucleotides containing a free sulphydryl group and subsequent attachment of thiol specific probes. *Nucleic Acids Res* **13**, 4485-4502 (1985).
8. Smyth, D. G., Blumenfeld, O. O. & Konigsberg, W. Reactions of N-ethylmaleimide with peptides and amino acids. *Biochem J* **91***,* 589-595 (1964).
9. Heitz, J. R., Anderson, C. D. & Anderson, B. M. Inactivation of yeast alcohol dehydrogenase by N-alkylmaleimides. *Arch Biochem Biophys* **127**, 627-636 (1968).
10. Podhradsky, D., Drobnica, L. & Kristian, P. Reactions of cysteine, its derivatives, glutathione coenzyme A, and dihydrolipoic acid with isothiocyanates. *Experientia* **35**, 154-155 (1979).
11. Vishnyakova, Golubeva & Glebova. *Russ. Chem. Rev.* **54**, 249-261 (1985).
12. Hazzard, Lammiman, Poon, Satchell & Satchell. *J. Chem. Soc., Perkin Trans. 2,* 1029 (1985).
13. Pieken, W. *et al.* (International Patent, 1998).
14. Mokhir, A. A., Tetzlaff, C. N., Herzberger, S., Mosbacher, A. & Richert, C. Monitored selection of dna-hybrids forming duplexes with capped terminal c:g base pairs. *J Comb Chem* **3**, 374-386 (2001).
15. Bischoff, R., Coull, J. M. & Regnier, F. E. Introduction of 5'-terminal functional groups into synthetic oligonucleotides for selective immobilization. *Anal Biochem* **164**, 336-344 (1987).
16. Ghosh, S. S. & Musso, G. F. Covalent attachment of oligonucleotides to solid supports. *Nucleic Acids Res* **15**, 5353-5372 (1987).
17. Allen, D. J., Darke, P. L. & Benkovic, S. J. Fluorescent oligonucleotides and deoxynucleotide triphosphates: preparation and their interaction with the large (Klenow) fragment of Escherichia coli DNA polymerase I. *Biochemistry* **28***,* 4601-4607 (1989).
18. Pitha, J., Kociolek, K. & Caron, M. G. Detergents linked to polysaccharides: preparation and effects on membranes and cells. *Eur J Biochem* **94**, 11-18 (1979).
19. Elling, L. & Kula, M. R. Immunoaffinity partitioning: synthesis and use of polyethylene glycol-oxirane for coupling to bovine serum albumin and monoclonal antibodies. *Biotechnol Appl Biochem* **13**, 354-362 (1991).
20. Wardell. in *The chemistry of the thiol group* (ed. Patai) 246-251 (Wiley, New York, 1974).
21. Zuckermann, R., Corey, D. & Schultz, P. Efficient methods for attachment of thiol specific probes to the 3'-ends of synthetic oligodeoxyribonucleotides. *Nucleic Acids Res* **15**, 5305-5321 (1987).
22. Teare, J. & Wollenzien, P. Specificity of site directed psoralen addition to RNA. *Nucleic Acids Res* **17**, 3359-3372 (1989).
23. Ghosh, S. S., Kao, P. M. & Kwoh, D. Y. Synthesis of 5'-oligonucleotide hydrazide derivatives and their use in preparation of enzyme-nucleic acid hybridization probes. *Anal Biochem* **178**, 43-51 (1989).
24. Ivanovskaya, M. G., Gottikh, M. B. & Shabarova, Z. A. Modifikation of oligo(poly)nucleotide phosphomonoester groups in aqueous solution. *Nucleosides Nucleotides* **6**, 913-934 (1987).
25. Ralph, R. K., Young, R. J. & Khorana, H. G. The labelling of phosphomonoester end groups in amino acid acceptor ribonucleic acids and its use in the determination of nucleotide sequences. *J. Am. Chem. Soc.* **84**, 1490-1491 (1962).
26. Chu, B. C., Wahl, G. M. & Orgel, L. E. Derivatization of unprotected polynucleotides. *Nucleic Acids Res* **11**, 6513-6529 (1983).
27. Shabarova, Z. A. Chemical development in the design of oligonucleotide probes for binding to DNA and RNA. *Biochimie* **70***,* 1323-34 (1988).

Die in der vorstehenden Beschreibung, den Ansprüchen sowie den, Zeichnungen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Modifizierte L-Nukleinsäure umfassend einen L-Nukleinsäure-Teil und einen Nicht-L-Nukleinsäure-Teil, wobei der L-Nukleinsäure-Teil mit dem Nicht-L-Nukleinsäure-Teil konjugiert ist und die Konjugation des L-Nukleinsäure-Teils mit dem Nicht-L-Nukleinsäure-Teil zu einer verlangsamten Ausscheidung aus einem Organismus führt, verglichen mit einer L-Nukleinsäure umfassend nur den L-Nukleinsäure-Teil.

2. Modifizierte L-Nukleinsäure umfassend einen L-Nukleinsäure-Teil und einen Nicht-L-Nukleinsäure-Teil, wobei der L-Nukleinsäure-Teil mit dem Nicht-L-Nukleinsäure-Teil konjugiert ist und die Konjugation des L-Nukleinsäure-Teils mit dem Nicht-L-Nukleinsäureteil zu einer erhöhten Verweildauer in einem Organismus führt, verglichen mit einer L-Nukleinsäure umfassend nur den L-Nukleinsäure-Teil.

3. Modifizierte L-Nukleinsäure, insbesondere nach Anspruch 1 und/oder Anspruch 2, umfassend einen L-Nukleinsäure-Teil und einen Nicht-L-Nukleinsäure-Teil, wobei der L-Nukleinsäure-Teil mit dem Nicht-L-Nukleinsäure-Teil konjugiert ist und der Nicht-L-Nukleinsäure-Teil ein Molekulargewicht von mehr als etwa 300 Da, bevorzugterweise mehr als etwa 20.000 Da und bevorzugtererweise mehr als etwa 40.000 Da aufweist.

4. Modifizierte L-Nukleinsäure nach einem der Ansprüche 1 bis 3, **dadurch gekennzeichnet, dass** die modifizierte L-Nukleinsäure ein Molekulargewicht von etwa 600 bis 500,000 Da, bevorzugterweise von etwa 10,000 bis 400,000 Da bevorzugtererweise von etwa 50,000 bis 300,000 Da aufweist.

5. Modifizierte L-Nukleinsäure nach einem der Ansprüche 1 bis 4, **dadurch gekennzeichnet, dass** der L-Nukleinsäure-Teil ein Molekulargewicht von 300 bis 50,000 Da, bevorzugterweise von 5,000 bis 25,000 Da bevorzugtererweise von 7,000 bis 15,000 Da aufweist.

6. Modifizierte L-Nukleinsäure nach einem der Ansprüche 1 bis 5, **dadurch gekennzeichnet, dass** der Nicht-L-Nukleinsäure-Teil an den L-Nukleinsäure-Teil über eine funktionale Gruppe des L-Nukleinsäure-Teils direkt oder indirekt verknüpft ist, die an einem der folgenden Bestandteile der L-Nukleinsäure vorhanden oder gebunden ist, wobei die funktionale Gruppe ausgewählt ist aus der Gruppe, die terminale und nicht-terminale Phosphate, terminale und nicht-terminale Zuckeranteile und natürliche und nicht-natürliche Purin- und natürliche und nicht-natürliche Pyrimidin-Basen umfasst.

7. Modifizierte L-Nukleinsäure nach Anspruch 6, **dadurch gekennzeichnet, dass** die Verknüpfung des Nicht-L-Nukleinsäure-Teils mit dem L-Nukleinsäure-Teil über die 2'-OH-, 3'-OH- und/oder 5'-OH-Gruppe oder eines Derivates davon eines oder mehrerer der Zuckeranteile des L-Nukleinsäure-Teils vorliegt.

8. Modifizierte L-Nukleinsäure nach Anspruch 6 oder 7, **dadurch gekennzeichnet, dass** die Verknüpfung über mindestens eine der Positionen 5 oder 6 der Pyrimidin-Base vorliegt.

9. Modifizierte L-Nukleinsäure nach einem der Ansprüche 6 bis 8, **dadurch gekennzeichnet, dass** die Verknüpfung über mindestens einer der Position 8 der Purin-Basen vorliegt.

10. Modifizierte L-Nukleinsäure nach einem der Ansprüche 6 bis 9, **dadurch gekennzeichnet, dass** die Verknüpfung an einer oder mehreren der exozyklischen und/oder endozyklischen Amingruppen und/oder Ketogruppen der Purin- und/oder Pyrimidin-Basen und/oder abasischen Position(en) vorliegt.

11. Modifizierte L-Nukleinsäure nach einem der Ansprüche 1 bis 10, **dadurch gekennzeichnet, dass** der Nicht-Nukleinsäure-Teil ausgewählt ist aus der Gruppe, die lineares Poly(ethylen)glykol, verzweigtes Poly(ethylen)glykol, Hydroxyethylstärke, Peptide, Proteine, Polysaccharide, Sterole, Polyoxypropylen, Polyoxyamidate, Poly(2-hydroxyethyl)-L-glutamin, precise Polyethylenglykol umfasst.

12. Modifizierte L-Nukleinsäure nach einem der Ansprüche 1 bis 11, **dadurch gekennzeichnet, dass** zwischen dem L-Nukleinsäure-Teil und dem Nicht-L-Nukleinsäure-Teil ein Linker angeordnet ist.

13. Modifizierte L-Nukleinsäure nach einem der Ansprüche 1 bis 12, **dadurch gekennzeichnet, dass** der L-Nukleinsäure-Teil eine Nukleinsäure umfasst gemäß SEQ IDNO. 1.

14. Modifizierte L-Nukleinsäure nach Anspruch 13, **dadurch gekennzeichnet, dass** der L-Nukleinsäure-Teil am 5'-OH-Ende als Linker 6-Aminohexylphosphat aufweist.

15. Modifizierte L-Nukleinsäure nach Anspruch 14, **dadurch gekennzeichnet, dass** an das freie Amin des Aminohexylphosphat-Linkers Polyethylenglykol gekoppelt ist.

16. Verwendung der modifizierten L-Nukleinsäure nach einem der Ansprüche 1 bis 15 als Diagnostikum.

17. Verwendung der modifizierten L-Nukleinsäure nach einem der Ansprüche 1 bis 15 zur Herstellung eines Medikamentes.

18. Verfahren zur Herstellung einer modifizierten L-Nukleinsäure umfassend einen L-Nukleinsäure-Teil und einen Nicht-L-Nukleinsäure-Teil, insbesondere einer modifzierten Nukleinsäure nach einem der Ansprüche 1 bis 15, **gekennzeichnet durch** die folgenden Schritte:
a) Bereitstellen einer L-Nukleinsäure, welchen den L-Nukleinsäure-Teil oder einen Teil davon der modifizierten L-Nukleinsäure ausbildet;
b) Bereitstellen einer Nicht-L-Nukleinsäure, die den Nicht-L-Nukleinsäure-Teil oder einen Teil davon der modifizierten L-Nukleinsäure ausbildet;
c) Umsetzen der L-Nukleinsäure aus a) und der Nicht-L-Nukleinsäure aus b); und
d) optional Isolieren der in Schritt c) erhaltenen modifizierten L-Nukleinsäure.

19. Verfahren nach Anspruch 18, **dadurch gekennzeichnet, dass** die L-Nukleinsäure in Schritt a) einen Linker umfasst.

20. Verfahren nach Anspruch 18, **dadurch gekennzeichnet, dass** nach dem Bereitstellen der L-Nukleinsäure in Schritt a) diese mit einem Linker versehen wird.
